(19) Europäisches Patentamt
European Patent Office
Office européen des brevets

(11) Publication number: **0 081 200**
**B1**

(12) # EUROPEAN PATENT SPECIFICATION

(45) Date of publication of patent specification: **08.10.86**

(51) Int. Cl.$^4$: **C 07 D 263/24,** A 61 K 31/42

(21) Application number: **82111135.8**

(22) Date of filing: **02.12.82**

(54) **p-Oxooxazolidinylbenzene sulfonamides as antibacterial agents.**

(30) Priority: **04.12.81 US 327583**
**15.09.82 US 417569**

(43) Date of publication of application:
**15.06.83 Bulletin 83/24**

(45) Publication of the grant of the patent:
**08.10.86 Bulletin 86/41**

(84) Designated Contracting States:
**AT BE CH DE FR GB IT LI LU NL SE**

**The file contains technical information submitted after the application was filed and not included in this specification**

(73) Proprietor: **E.I. DU PONT DE NEMOURS AND COMPANY**
**1007 Market Street**
**Wilmington Delaware 19898 (US)**

(72) Inventor: **Gregory, Walter Adelman**
**104 Rockingham Drive**
**Wilmington, DE 19803 (US)**

(74) Representative: **von Kreisler, Alek, Dipl.-Chem. et al**
**Deichmannhaus am Hauptbahnhof**
**D-5000 Köln 1 (DE)**

(56) References cited:
**EP-A-0 050 827**
**FR-A-2 458 547**
**GB-A-2 003 151**
**US-A-3 456 241**
**US-A-4 128 654**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 081 200 B1

(56) References cited:

**W.KERN: "Hagers Handbuch der Pharmazeutischen Praxis", vol. 2, 1969, Fourth New Edition, Editors P.H.List**

**BURGER'S MEDICINAL CHEMISTRY, 4th Edition, Part 1, 1980, p.21, 129**

## Description

Background of the Invention

In W. Kern, "Hagers Handbuch der pharmazeutischen Praxis", Vol. 2, pages 519 to 534 (1969) sulfonamides are described to be compounds in which not only the nitrogen atom of the sulfonamide group but also the nitrogen atom of the aromatic amino group is bearing substituents. It is further disclosed therein that the mechanism of action of sulfonamides is seen to be the competitive action of these compounds to p-aminobenzoic acid at the active site of the enzym dihydropteroate synthetase. This view of the action of the commonly known anti microbial sulfonamides and sulfones is also supported by "Burger's Medicinal Chemistry" 4th edition, part I (1980), pages 21 to 31. By the same document it has been contributed that disubstitution at the aromatic aminonitrogen atom in general leads to inactive compounds. Furthermore, the primary amino group at the aromatic ring in sulfonamides apparently plays a vital part in producing bacteriostasis, since any substituent on it causes complete lack of activity. In other words: sulfonamides (di-)substituted at the aromatic ring nitrogen atom cause a loss of bacteriostatic activity of the sulfonamide. This is, in particular, the case if substituents at that nitrogen atom cannot readily be removed in vivo.

U.S. Patent 4,128,654 to Fugitt et al. discloses, among others, compounds of the formula:

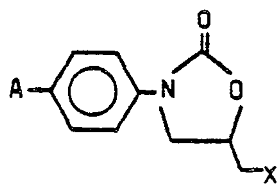

where

$A = RS(O)_n$;
X = Cl, Br or F;
$R = C_1$—$C_3$ alkyl; and
N = 0, 1 or 2.

The compounds are disclosed as being useful in controlling fungal and bacterial diseases of plants.

U.K. Patent 2003—151 to Delande teaches the following compound as an antidepressant:

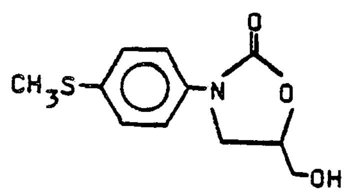

U.S. Patent 4,340,606 to Fugitt et al discloses antibacterial agents of the general formula:

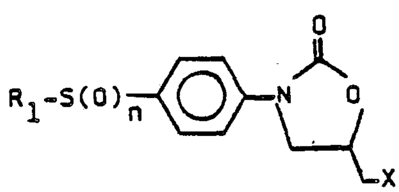

where

$R_1 = CH_3$, $C_2H_5$, $CF_2H$, $CF_3$ or $CF_2CF_2H$; and
$X = OR_2$.

Summary of the Invention

This invention relates to novel compounds of Formula I.

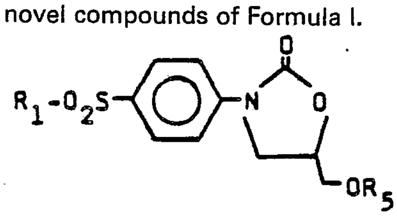

(I)

where

$R_1$ is $-NR_2R_3$, $-N(OR_2)R_3$, $-N_3$, $-NHNH_2$, $-NX_2$, $-NR_{10}X$, $-NXZ$ or $-N{=}S(O)_nR_8R_9$;

$R_2$ and $R_3$ are independently H, alkyl of 1—4 carbons or cycloalkyl of 3—8 carbons;

$R_5$ is

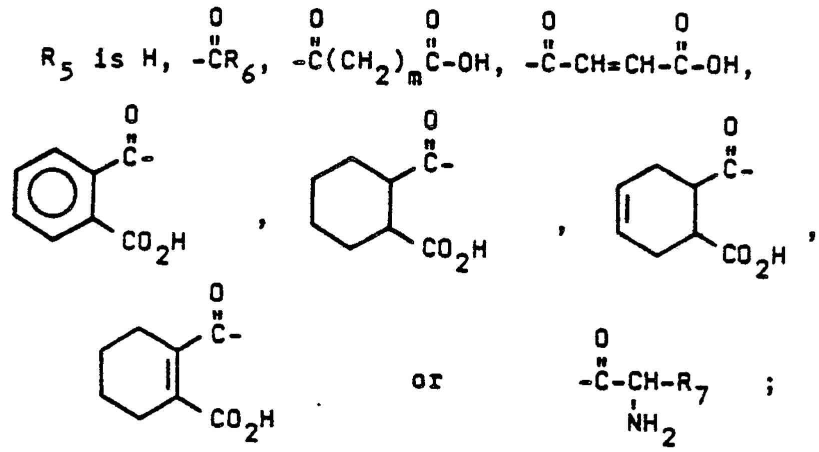

or

$R_6$ is aryl or alkyl of 1—12 carbons;

$R_7$ is H, alkyl of 1—5 carbons, $-CH_2OH$, $CH_2SH$, aryl or aralkyl;

$R_8$ and $R_9$ are independently alkyl of 1—4 carbons or, taken together are $-(CH_2)_p-$;

$R_{10}$is alkyl of 1—4 carbons;

X is Cl or Br;

Z is a physiologically acceptable cation;

m is 2 or 3;

n is 0 or 1; and

p is 3, 4 or 5;

and pharmaceutically acceptable salts thereof.

Test results indicate that compounds of Formula I are useful for alleviating bacterial infections in mammals. This invention therefore also relates to pharmaceutical compositions containing compounds of Formula I and the use of those same compounds for the manufacture of a medicament to alleviate bacterial infection in mammals.

Preferred because of their high antibacterial activity are those compounds where, independently, $R_5$ is H and $R_1$ = $NH_2$ or $NH-CH_3$. Specifically preferred for its high antibacterial activity is the comopound (l)-4-[5-(hyroxymethyl)-2-oxooxazolidin-3-yl]benzenesulfonamide.

The term "aryl" as used in the definitions of substituents $R_6$ and $R_7$ is meant to encompass any univalent aromatic radical, either homocyclic or heterocyclic. Suitable aryl groups include radicals of monocylic compounds such as benzene, pyridine, pyrimidine, pyrazole, furan, triazine, thiophene, imidazole, oxazole, thiazole and pyrrole. Radicals of polycyclic compounds such as biphenyl and terphenyl as well as of condensed polycyclic compounds such as naphthalene, anthracene, phenanthrene, quinoline, isoquinoline, acridine, phenazine, indole, benzothiophene, carbazole and dibenzofuran are also suitable.

Any of the aryl groups may optionally be substituted at any position with one or more substituents, including, but not limited to, F, Cl, Br, $NO_2$, $C_1-C_3$ alkyl or alkoxy, OH, $CF_3$, CN, $CO_2(C_1-C_3$ alkyl) or $S(O)_qCH_3$ where q = 0, 1 or 2. The preferred aryl substituents are pyridine, thiophene, furan, pyrrole and benzene, optionally substituted with the abovementioned subsitutents.

The term "aralkyl" used in the definition of $R_7$ is meant to encompass an alkyl substituent, preferably containing one to four carbon atoms, substituted with any one of the aryl substituents previously described. Physiologically acceptable cations (Z) include alkali or alkaline earth metal ions such as K+, Mg++, Ca++, Li+ and Na+. Other suitable ions would be known to one skilled in the art.

Detailed Description of the Invention

Synthesis

Compounds of Formula I where $R_5$ = H and $R_1$ = $NR_2R_3$, or $N(OR_2)R_3$ represented by Formula Ia, can be prepared as illustrated in Scheme 1 in a manner known per se.

Scheme 1:

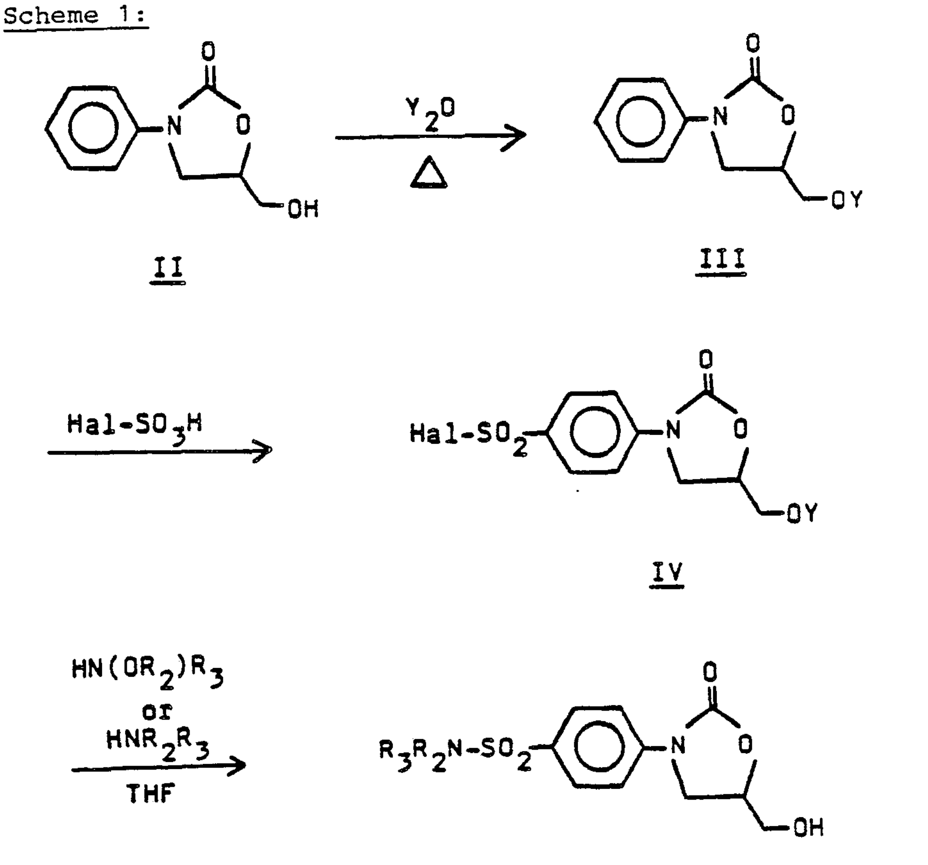


II

III

IV

Ia ($R_2$=$R_2$ or $OR_2$)

where Y is an acyl group.

The —OH function of 5-hydroxymethyl-3-phenyl-2-oxazolidinone (II) is blocked by refluxing this compound with a reagent which will introduce an acyl group, preferably an alpha-halogenated alkanoyl group. Suitable reagents for this purpose include trifluoroacetic anhydride, dichloroacetyl chloride, dichloroacetic anhydride, trichloroacetyl chloride, and trichloroacetic anhydride. The mixture is refluxed at a temperature in the range of about 0° to 150°C, preferably 0° to 40°C. This step may be done in the absence of solvent or with any inert hydrocarbon or chlorinated hydrocarbon solvent. The resulting ester III is contacted with either a halosulfonic acid such as chlorosulfonic acid or fluorosulfonic acid at a temperature of about 30 to 40°C to yield the sulfonyl halide of Formula IV. This step also may be done in the absence of solvent or in chlorinated hydrocarbon solvents such as $CHCl_3$, $CH_3CCl_3$ or $CCl_4$. This product is then dissolved in a solvent such as tetrahydrofuran, ether, dioxane, 1,2-dimethoxyethane, dimethylformamide, dimethylacetamide or urea solvents, and contacted with the appropriate amine, $HNR_2R_3$, or $HN(OR_2)R_3$, at a temperature in the range of about −20°C to 30°C, to yield the compound of Formula Ia.

Compounds of Formula I where X = OH and $R_1$ = $N_3$, represented by Formula Ib, can be preapred in a manner known per se by contacting the sulfonyl halide IV with sodium or potassium azide in an acetone-water mixture, as illustrated in Scheme 2. Suiable solvents include acetone, lower boiling ketone solvents such as methyl ethyl ketone, diethyl ketone and cyclopentanone, ether solvents and dipolar aprotic solvents.

Scheme 2:

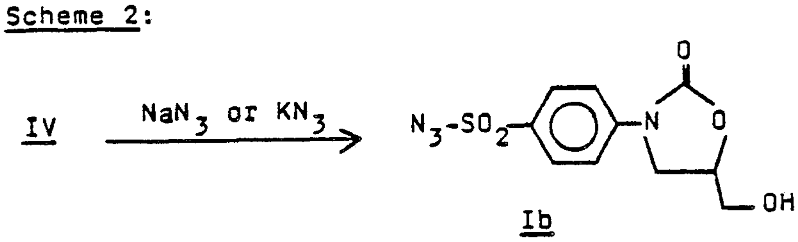


Ib

The azides Ib as well as the hydrazides Ic of this invention can also be prepared according to Scheme 3 in a manner known per se.

Scheme 3:

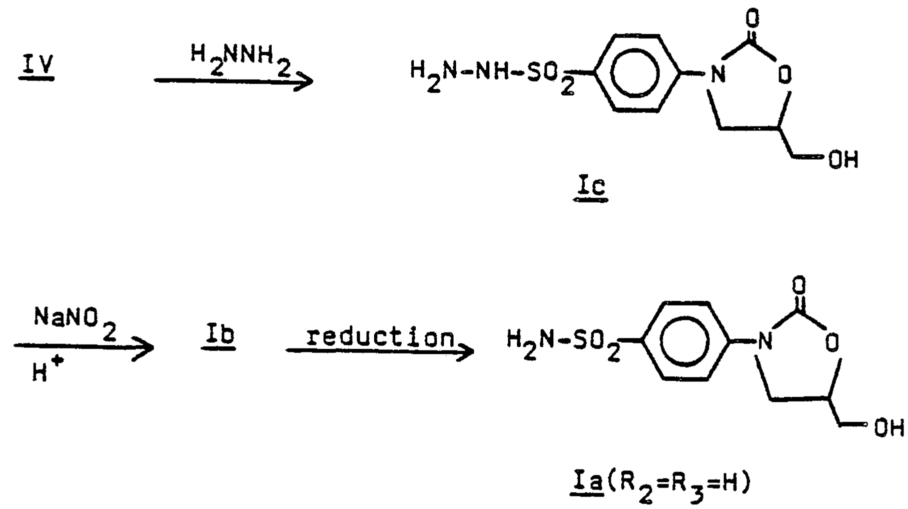


The sulfonyl halide IV is contactd with hydrazine to prepare the hydrazide Ic. This can then be reacted with a nitrite such as sodium nitrite in dilute acid solution to yield the azide Ib. The azide Ib can be reduced by any one of several known methods to yield the sulfonamide Ia. Suitable reduction methods include catalytic methods, reaction with sodium borohydride in an alcohol or in tetrahydrofuran or reaction with zinc and acetic acid.

Compounds of the invention where $R_5$=OH and $R_1$=NXZ, $NX_2$ or N=S(O)$_n$$R_8R_9$ can be prepared in a manner known per se as illustrated in Scheme 4:

Scheme 4:

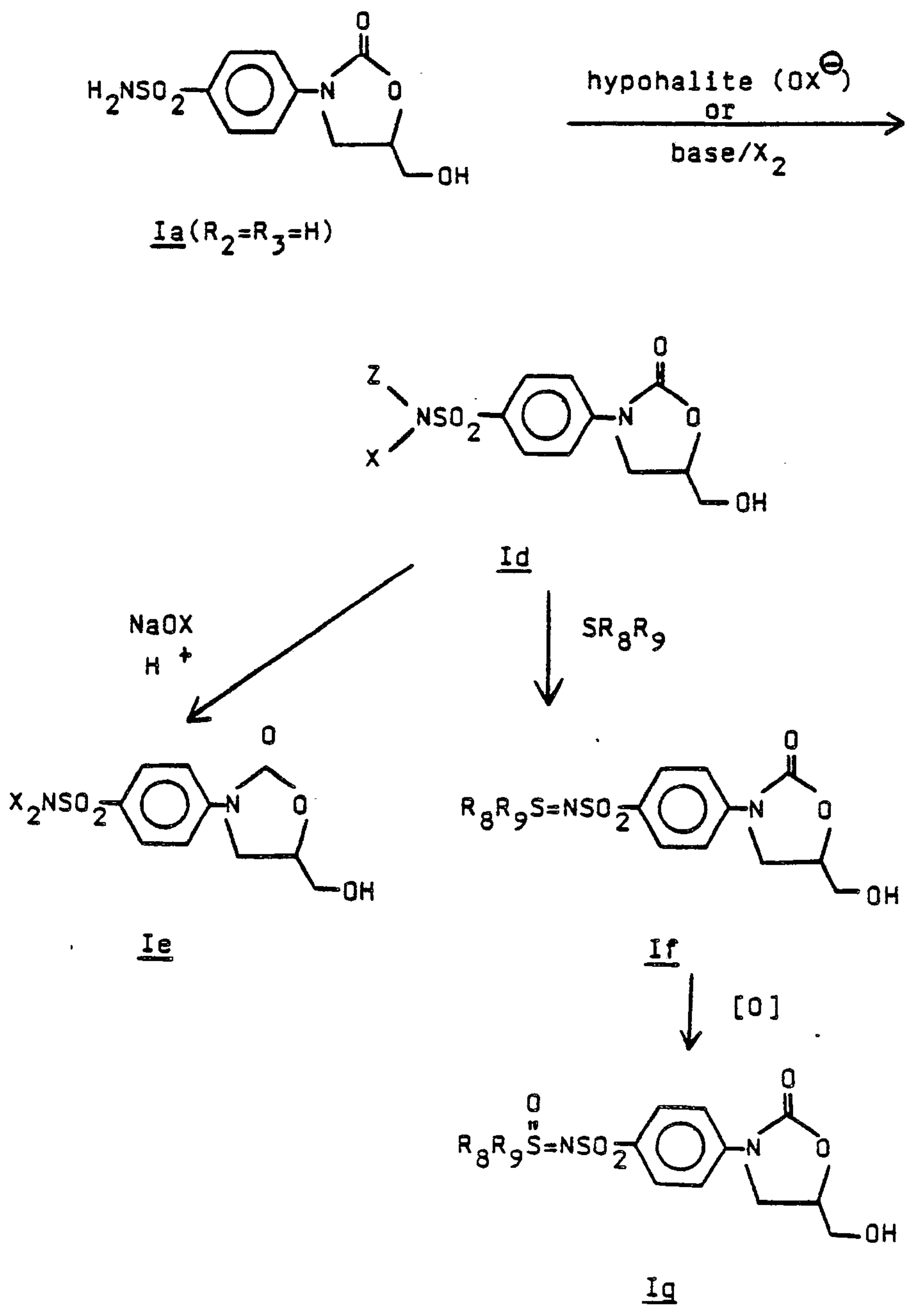


The halosulfonamide Id can be readily prepared by contacting a sulfonamide Ia with halogen and a base such as sodium hydroxide or with a preformed hypohalite such as sodium hypochlorite or calcium hypochlorite. To prepare the N,N-dihalosulfonamide Ie, the pH is adjusted to the range of about 4 to 8. Methods of adjusting the pH include addition of acetic acid, carbon dioxide or dilute mineral acid.

The sulfilimines If can be prepared in a manner known per se by contacting the halosulfonamide Id with a dialkyl sulfide, $SR_8R_9$ such as dimethylsulfide, trimethylene sulfide, methylethyl sulfide or tetrahydrothiophene, preferably in a water-alcohol solution. Oxidation of the sulfilimines with, for example, hypochlorite or meta-chloroperbenzoic acid, yields the sulfoximines Ig.

Compounds of the invention where $R_1=NR_{10}X$ can be prepared in a manner known per se, as illustrated in Scheme 5, by reaction with a hypohalite.

Scheme 5:

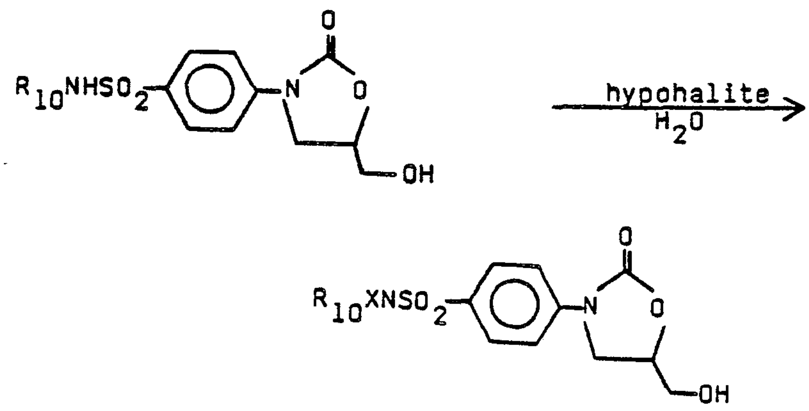


Ih

Various simple esters of this invention, represented by Formula Ii, can be prepared — in a manner known per se — as shown in Scheme 6 by reaction of a compound of Formula Ia—Ih with the appropriate acid chloride or anhydride, preferably in the presence of a base such as pyridine or 4-dimethylaminopyridine. The preferred solvent is pyridine or other solvents of the pyridine class, although in many cases no solvent is required.

Scheme 6:

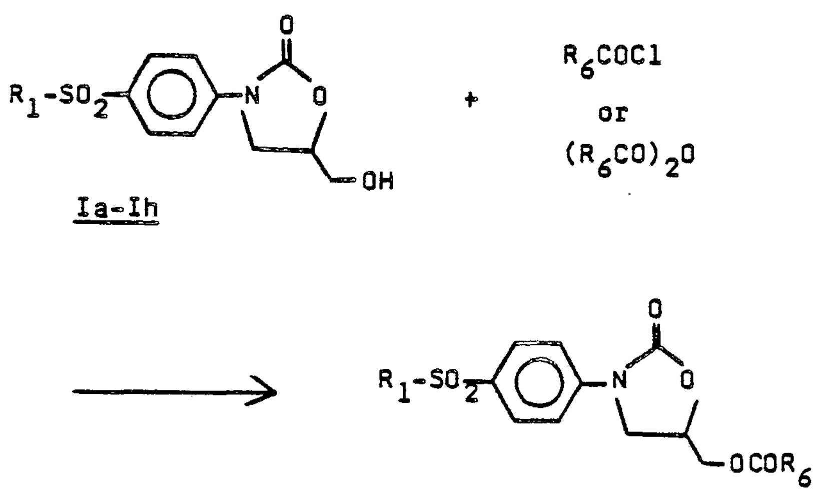


Ia-Ih

Ii

Many of the simple esters can also be prepared in a manner known per se by halosulfonating a compound of Formula V as illustrated in Scheme 7. No solvent is required, but solvents inert to the halosulfonic acid may be used. The product sulfonyl halide VI is then reacted with ammonia or an appropriate amine or an azide or hydrazine as in Schemes 1, 2, and 3 to yield the ester of Formula Ij.

Scheme 7:

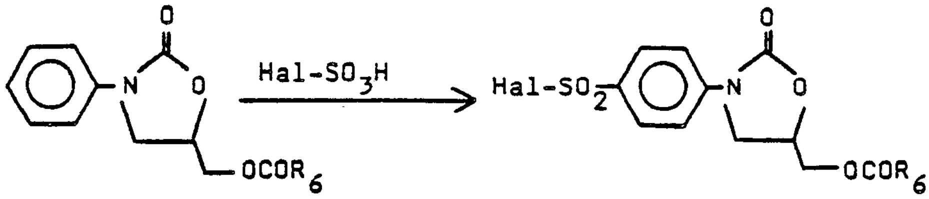


V VI

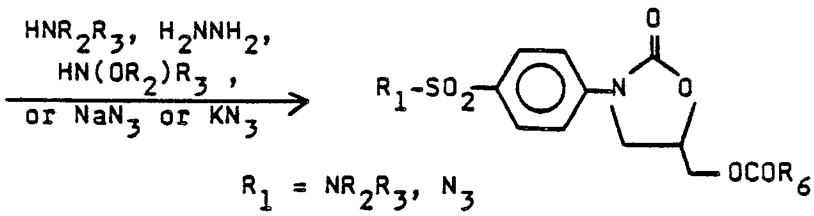


$R_1$ = $NR_2R_3$, $N_3$
$NHNH_2$ or
$N(OR_2)R_3$

Ij

The starting material V can be prepared — in a manner known per se — from 5-hydroxymethyl-3-phenyl-2-oxazolidinone as shown in Scheme 8.

Scheme 8

II 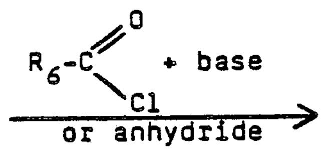 V


Other esters of this invention, Ik, can be prepared — in a manner known per se — as illustrated in Scheme 9, by contacting an alcohol with an appropriate anhdyride:

Scheme 9:

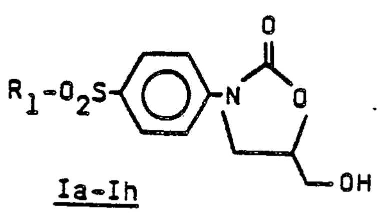

Ia-Ih

+

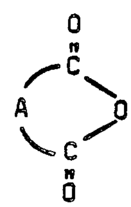

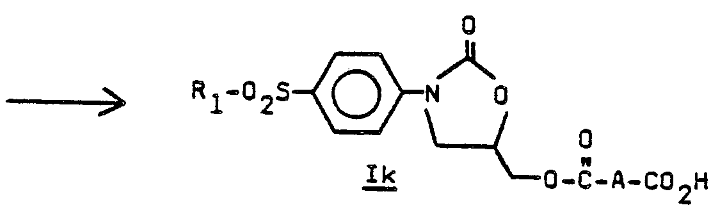

Ik

where

A = $-CH=CH-$, $-(CH_2)_m-$, 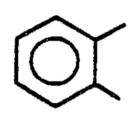,

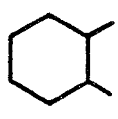, 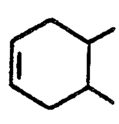 or 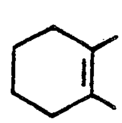;

and m is 2 or 3.

Suitable solvents are weak bases of the pyridine class and a suitable temperature range for the reaction is about 30 to 115°C.

The products obtained by the methods described above (Ia-Im) are racemic mixtures, that is, 50:50 mixtures of (+) and (−) enantiomers. The synthesis of optically active compounds can be achieved by using as a starting material in Scheme 1 the compound *l*-5-hydroxymethyl-3-phenyl-2-oxazolidinone (IIa). This starting material can be obtained by the desulfurization of *l*-3-[(4-methylthio)phenyl]-5-hydroxymethyl-2-oxazolidinone (VII) as shown in Scheme 10.

Scheme 10:

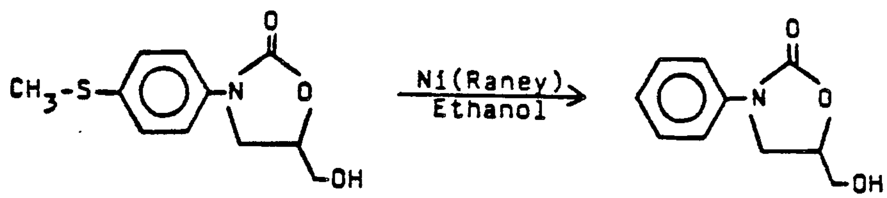


l-isomer    l-isomer.

VII    IIa

A preferred process for preparing l-5-hydroxymethyl-3-phenyl-2-oxazolidinone (IIa) is illustrated in Scheme II.

Scheme 11:

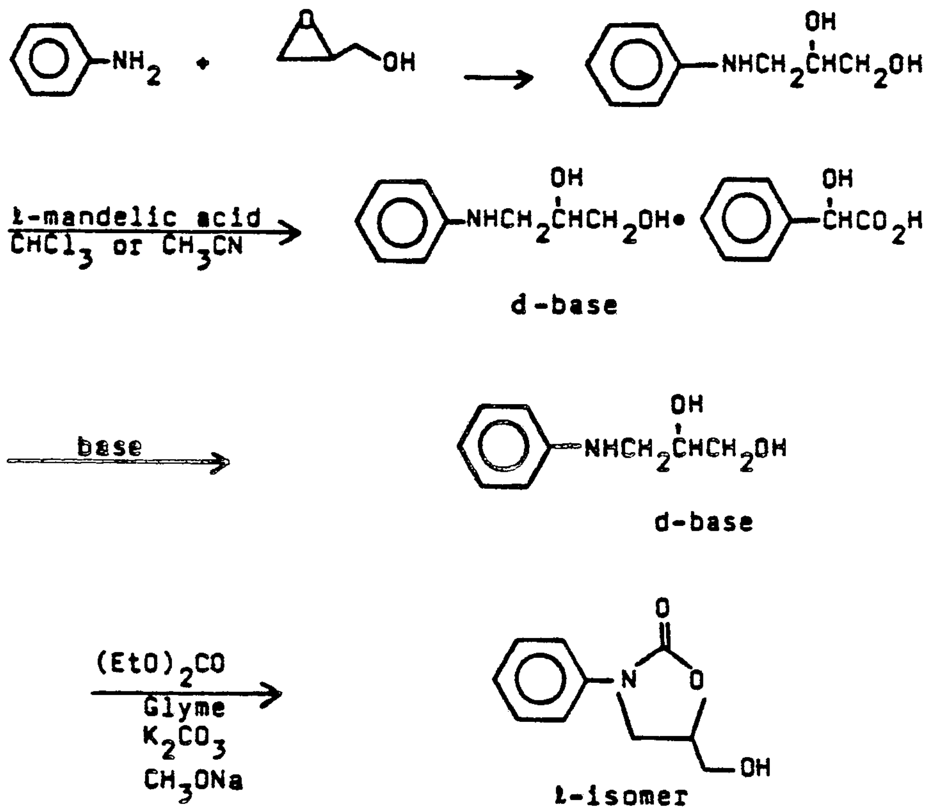


d-base

d-base

l-isomer

IIa

Aniline in a 2 to 10 molar excess is reacted with glycidol. The excess aniline is removed by distillation, and the (dl)-3-phenylamino-1,3-propanediol is distilled under reduced pressure. It is resolved by combining it with about 50—60 mole percent l-mandelic acid in chloroform or acetonitrile. The desired salt crystallizes and may be purified by recrystallization from chloroform or acetonitrile. The (d)-3-phenyl-amino-1,3-propanediol can be isolated by contacting it with a base. The salt is suspended in water, the pH of the mixture is brought to between 8 and 10, the mixture is then saturated with sodium chloride, and then is

continuously extracted with dichloromethane. Alternatively, the salt is dissolved in a water-alcohol mixture and passed over a strongly basic ion exchange resin. In either case, the resulting extract or column eluate is concentrated to leave the (d)-3-phenylamino-1,3-propanediol. This product is then reacted with diethyl carbonate (other carbonates such as dimethyl carbonate or diphenyl carbonate may be used) in a polar solvent such as 1,2-dimethoxyethane using potassium carbonate and sodium methoxide as catalysts. The product is isolated by concentrating the solvent, diluting with water and a little acetic acid, and washing with water. The product may be purified by recrystallization from 95% ethanol or acetonitrile. By using d-mandelic acid in this process, one can obtain the d-isomer of 5-hydroxymethyl-3-phenyl-2-oxazolidinone.

Another route to optically active compounds of the invention consists of carrying out the first reaction in Scheme II using optically active glycidol, preferably R-glycidol, to provide the optically active base directly.

The l-5-hydroxymethyl-3-phenyl-2-oxazolidinone (IIa) may also be prepared from (R)-(+)-1-benzylglycerol [S. Takano, E. Goto, M. Hirama, and Ogasawara, *Heterocycles 16,* 381 (1981)] followed by tosylation with p-toluene-sulfonyl chloride in pyridine and reacting this (S) tosyl ester (VIII) with the potassium salt of N-phenyl-4-methylbenzenesulfonamide in a dipolar aprotic solvent such as DMF. The resulting product (IX) is reacted with sodium naphthalene radical anion to produce product (X) which is reacted with diethyl carbonate or other carbonate ester in the presence of a catalytic amount of sodium methoxide to produce the desired l-isomer. This sequence is illustrated in Scheme 12.

Scheme 12:

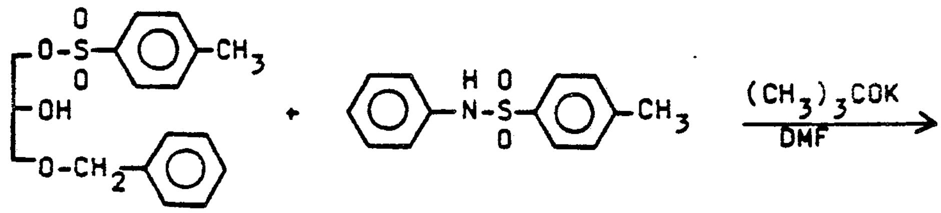


(S)-isomer

VIII

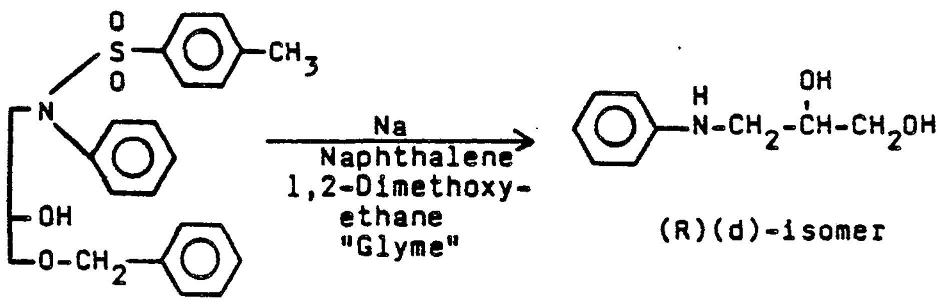


(R)(d)-isomer

(R)-isomer

IX

X

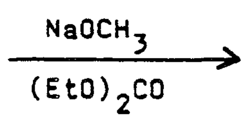


IIa

Amino acid esters of this invention ($R_5$=COCH($NH_2$)$R_7$) may be prepared by the reaction sequence shown in Scheme 13.

Scheme 13:

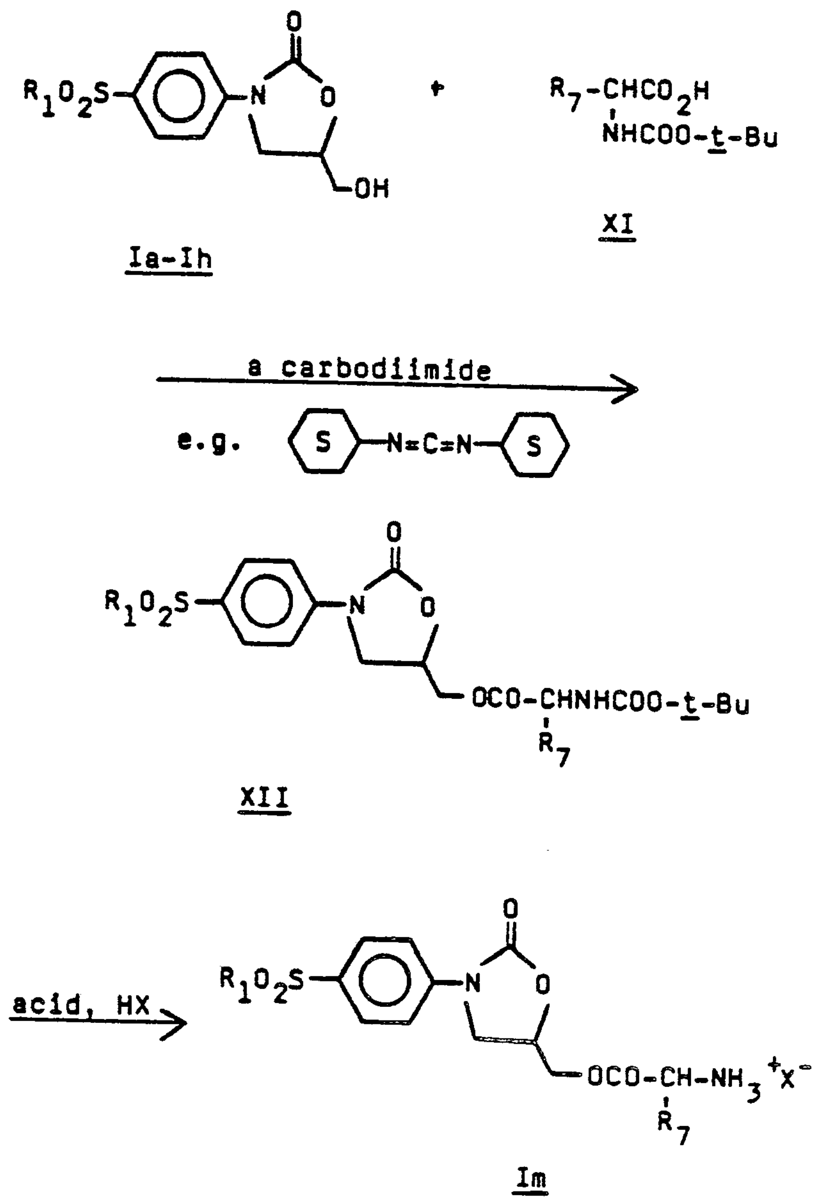


Ia-Ih

XI

XII

Im

An alcohol is contacted with the appropriate blocked amino acid (XI) in the presence of a carbodiimide such as dicyclohexyl carbodiimide. Suitable solvents include dichloromethane, chloroform, tetrahydrofuran and ether, to which may be added a small amount of a weak base such as pyridine. Suitable temperatures are in the range of about 0 to 100°C. The filtered product (XII) is treated with a volatile acid (e.g., trifluoroacetic acid) at 0—50°C. Upon removal of the acid by, for example, vacuum distillation, the ester Im in the form of its acid salt (e.g., trifluoroacetic acid salt) remains. This may be freed as an amine by treatment with a base. Treatment of this amine with an appropriate acid yields desired salts such as hydrochloride, phosphate, sulfate, acetate and benzoate salts.

The acidic compounds (Ik) of this invention form salts with various inorganic and organic bases which salts are within the scope of this invention. Such salts include alkali metal salts such as sodium and potassium salts, ammonium salts, alkaline earth metal salts such as calcium and magnesium salts, salts with organic bases such as the lower aliphatic amines, benzylamine, dicyclohexylamine, and salts with basic amino acids such as arginine.

Example 1

4-[5-(Hydroxymethyl)-2-oxooxazolidin-3-yl]benzenesulfonamide ($R_1$ = $NH_2$, $R_5$ = H)

A solution of 247.3 g (1.00 mole) of N-phenyl-4-methylbenzenesulfonamide containing 20 g of 1,4-diazabicyclo[2.2.2]octane (DABCO) in 950 ml dimethylformamimde (DMF) was stirred and heated under nitrogen as a solution of 70 ml of freshly distilled glycidol in 70 ml of DMF was added over a period of two hours. Heating was continued for 1 hour and 40 minutes, and then a further 35 ml of glycidol in 35 ml of DMF was added over a period of one hour. Heating was continued for 5/12 hours. The reaction mixture was poured into 4 l of cold water (a little ice present) and the product, N-(2,3-dihydroxypropyl)-4-methyl-N-phenylbenzenesulfonamide, crystallized. This was filtered and washed well with cold water to yield 348 g, m.p. 91—94°C, and was then recrystallized from 700 ml of toluene to yield 312 g, m.p. 108.5—109.5°C.

250 Grams of 40% sodium dispersion in mineral oil was added to a solution of 600 g of naphthalene in 1.8 l of 1,2-dimethoxyethane ("Glyme") stirred under nitrogen and maintained at a temperature between 20° to 30°C. After all the sodium was added, the mixture was stirred 20 minutes, and then 305 g of N-(2,3-dihydroxypropyl)-4-methyl-N-phenylbenzenesulfonamide was added through a powder addition funnel while maintaining the temperature of the reaction mass below 35°C. After all of the solid was added, the mixture was stirred one hour. Water was then added until the color of the mixture faded from dark green or black to yellow; then concentrated hydrochloric acid was added until the solution was strongly acidic. The mixture was extracted twice with toluene and then three times with hexane, was sparged with nitrogen to remove the hexane, saturated with sodium chloride and then made basic with concentrated ammonium hydroxide. The product was extracted with dichloromethane; and the extract was dried over potassium carbonate. The filtered dichloromethane was concentrated to yield 132.3 g of 3-phenylamino-1,2-propanediol, a pale yellow oil.

A mixture of 83.6 g (0.5 mole) of 3-phenylamino-1,2-propanediol, 250 ml of 1,2-dimethoxyethane and 61 ml of diethyl carbonate was refluxed in a nitrogen atmosphere. Solid sodium methoxide (about 0.15 g) was added and refluxing continued for approximately 2½ hours. The mixture was cooled, stirred with water and filtered to yield 41.1 g of 5-(hydroxymethyl)-3-phenyl-2-oxazolidinone, m.p. 122—124°C. The 41.1 g was recrystallized from 100 ml of absolute ethanol to give 38.0 g, m.p. 125.5—126°C. A further crop of 25.3 g (m.p. 124.5—125°C) was obtained from the water filtrate by concentrating and recrystallizing from ethanol.

A mixture of 25.3 g of 5-(hydroxymethyl)-3-phenyl-2-oxazolidinone and 100 g of trifluoroacetic anhydride was refluxed under $N_2$ for one hour. The solid all dissolved. The mixture was then concentrated under reduced pressure to give 41 g of oil which crystallized on standing. This product was used without further purification.

About 250 ml of chlorosulfonic acid was stirred well in a flask under nitrogen, as the above product was added over fifteen minutes. The mixture was not cooled and the temperature stayed between 30—40°C. The solid dissolved with evolution of heat and hydrogen chloride. The mixture was stirred at ambient temperature for one hour and twenty minutes and was then quenched on ice. The product (dl)-4-[5-(trifluoroacetoxymethyl)-2-oxooxazolidin-3-yl]benzenesulfonyl chloride, crystallized and was filtered and washed well with cold water and dried in a nitrogen stream. The product was then dissolved in tetrahydrofuran and stirred in an ice bath as 18 ml of concentrated ammonium hydroxide was added, keeping the temperature between 20—25°C. Concentration and filtration yielded 24 g of the title compound, m.p. 154—156°C. This was recrystallized from acetonitrile to give 15.8 g, m.p. 167—168°C.

Example 2

*l*-4-[5-(Hydroxymethyl)-2-oxooxazolidin-3-yl]benzenesulfonamide ($R_1$ = $NH_2$, $R_5$ = H)

300 Grams of Raney nickel catalyst was added with stirring, to a solution of 40.4 g (0.169 mole) *l*-3-[(4-methylthio)phenyl]-5-hydroxymethyl-2-oxazolidinone in 500 ml absolute ethanol, and the resulting mixture was refluxed for one hour. A sample of the reaction mixture was checked by NMR which indicated removal of the —$SCH_3$ group. The solution was then filtered and the reaction mixture extracted repeatedly with boiling ethanol. The alcohol extracts were combined and concentrated to yield 32.5 g of *l*-5-hydroxymethyl-3-phenyl-2-oxazolidinone, m.p. 134—136°C. This product was recrystallized from 115 ml absolute ethanol to give 32.5 g of white crystals melting at 138—139.5°C. This product was recrystallized once more than 106 ml absolute ethanol to yield 28.9 g of product, m.p. 139—140°C.

A mixture of 28.7 g (0.148 mole) *l*-5-hydroxymethyl-3-phenyl-2-oxazolidinone in 100 ml trifluoroacetic anhdyride was refluxed until the solid dissolved. This mixture was concentrated under reduced pressure, and the resulting oil was added to 200 ml chlorosulfonic acid, while maintaining the mixture at a temperature below 30°C. The mixture was then stirred at ambient temperature for 2¼ hours and then poured over ice. The product which crystallized (l-4-[5-(trifluoroacetoxymethyl)-2-oxooxazolidin-3-yl]benzenesulfonyl chloride) was filtered and washed with water and then added to a mixture of 60 ml concentrated ammonia in 300 ml THF, maintaining the temperature of the resulting mixture at −10° to 0°C. The resulting mixture was stirred for 15 minutes at 0°C and for an additional 30 minutes without external cooling. The mixture was then concentrated under reduced pressure to remove THF and diluted with water. The product was washed with water and filtered to yield 32.1 g of the title compound, m.p. 182—184°C. (A change of crystal form was noted at 158°C). This was recrystallized from acetonitrile to give 25.5 g, m.p. 184.5—185°C.

Example 3

(dl)-N,N-Dimethyl and (dl)-N-Methyl-4-[5-(hydroxymethyl)-2-oxooxazolidin-3-yl]benzenesulfonamide ($R_1$ = $N(CH_3)_2$ or $NHCH_3$, $R_5$ = H)

A solution of 2.72 g (0.01 mole) (dl)-[5-(hydroxymethyl)-2-oxooxazolidin-3-yl]benzenesulfonamide in 50 ml dry dimethylformamide (DMF) containing 5 g anhydrous potassium carbonate was stirred at 60°C as 0.62 ml iodomethane in 10 ml DMF was added. Following this addition, the mixture was kept at 60°C for one hour and was then concentrated under reduced pressure to distill out the DMF. The residue (3.7 g) was taken up in dimethyl sulfoxide and chromatographed on silica gel to give an early cut of 0.65 g (dl)-N,N-dimethyl-4-[5-(hydroxymethyl)-2-oxooxazolidin-3-yl]benzenesulfonamide, m.p. 174—176°C. A second cut of 1.05 g (dl)-N-methyl-4-[5-(hydroxymethyl)-2-oxooxazolidin-3-yl]benzenesulfonamide, m.p. 164—166°C was also obtained.

Example 4

(dl)-4-[5-(Hydroxymethyl)-2-oxooxazolidin-3-yl]benzenesulfonyl Azide ($R_1$ = $N_3$, $R_5$ = H)

A solution of 10 g sodium azide in 20 ml water was added to 200 ml reagent grade acetone and then cooled to −5°C. A 19 g portion of (dl)-4-[5-(trifluoroacetoxymethyl)-2-oxooxazolidin-3-yl]benzenesulfonyl chloride (prepared as in Example 1) was added to the solution while maintaining the temperature of the mixture below 0°C. After stirring the mixture for one hour, it was allowed to stand overnight. The acetone was removed under reduced pressure, water was added and the product was filtered and washed with water to yield 12.8 g, m.p. 108—110°C. This product was stirred with 250 ml acetonitrile at 25°C and filtered to remove some insoluble material. The acetonitrile was concentrated and the crystallized product filtered to yield 12.3 g. This product was recrystallized from ethyl acetate to yield 6.0 g of the title compound, m.p. 112—113°C.

Example 5

l-4-[5-(Hydroxymethyl)-2-oxooxazolidin-3-yl]benzenesulfonyl Azide ($R_1$ = $N_3$, $R_5$ = H)

A solution of 5 g sodium azide in 10 ml water was added to 100 ml reagent grade acetone and then cooled to −5°C. A 7 g portion of l-4-[5-(trifluoroacetoxymethyl)-2-oxooxazolidin-3-yl]benzenesulfonyl chloride (prepared as in Example 2) was added, while maintaining the temperature of the mixture between −5 to 0°C. After stirring the mixture for one hour at 0°C and allowing it to stand overnight, the acetone was removed under reduced pressure. Water was added and the solid was filtered and washed with water to yield 4.8 g, m.p. 136—138°C. This solid was recrystallized from ethyl acetate to yield 4 g of the title compound, m.p. 137—138°C.

Example 6

l-4-[5-(Hydroxymethyl)-2-oxooxazolidin-3-yl]-N-methylbenzenesulfonamide ($R_1$ = $NHCH_3$, $R_5$ = H)

A solution of 10 ml methylamine in 200 ml dry tetrahydrofuran (THF) was stirred as 6.3 g *l*-4-[5-(trifluoroacetoxymethyl)-2-oxooxazolidin-3-yl]benzenesulfonyl chloride was added. The mixture was maintained at a temperature of −10°C or below during this addition and was then allowed to come to ambient temperature while being stirred overnight. After removing the THF under reduced pressure, water was added and a white solid was filtered off and washed with water to yield 4.41 g of product, m.p. 137—142°C. Recrystallization from acetonitrile yielded 3.83 g of the title compound, m.p. 137—138°C.

Example 7

Using the procedure in Example 6, one may prepare (*l*)-4-[5-(hydroxymethyl)-2-oxooxazolidin-3-yl]-N-*n*-butylbenzenesulfonamide ($R_1$ = NH-*n*-$C_4H_9$, $R_5$ = H) by using 4 ml of *n*-butylamine in place of the methylamine.

Example 8

Using the procedure in Example 6, one may prepare (*l*)-N,N-*n*-dibutyl-4-[5-(hydroxymethyl)-2-oxooxazolidin-3-yl]benzenesulfonamide ($R_1$ = N(*n*-$C_4H_9)_2$, $R_5$ = H) by using 5.5 ml of di-*n*-butylamine in place of the methylamine.

Example 9

l-4-[5-(Hydroxymethyl)-2-oxooxazolidin-3-yl]-N-cyclopropylbenzenesulfonamide ($R_1$ = NH—◁, $R_5$ = H)

A solution of 6 ml cyclopropylamine in 50 ml tetrahydrofuran was stirred in an ice bath, keeping the temperature below 30°C, as 9.3 g of *l*-4-[5-(trifluoroacetoxymethyl)-2-oxooxazolidin-3-yl]benzenesulfonyl chloride (see Example 2) was added. The mixture was stirred one hour, the tetrahydrofuran was removed under reduced pressure and the residue was diluted with water and made acid with dilute hydrochloric acid. The solid was filtered, washed with water and dried. The yield of the title compound was 7.06 g, m.p. 129—147°C. This was crystallized from 30 ml acetonitrile to give 2.0 g, m.p. 161.8—163.4°C.

Example 10

*l*-4-[5-(Hydroxymethyl)-2-oxooxazolidin-3-yl]-N-methoxy-benzenesulfonamide ($R_1$ = $NHOCH_3$, $R_5$ = H)

A solution of 3 ml methoxyamine (O-methylhydroxylamine) in 50 ml tetrahydrofuran was stirred and cooled to −5 to 10°C as a solution of 4.4 g. *l*-4-[5-(Trifluoroacetoxymethyl)-2-oxooxazolidin-3-yl]benzenesulfonyl chloride (see Example 2) in 20 ml tetrahydrofuran was added over a 15 minute period. A white solid separated. The mixture was allowed to warm to ambient temperature and was filtered and concentrated to give a colorless gum. This (4.5 g) was chromatographed using the Water Co. Prep-500 using a 45% acetonitrile; 55% dichloromethane solvent mixture. A fifth cut (1.8 g) was crystallized from ethyl acetate to give 1.04 g of the title compound, m.p. 129.6—131.5°C. This was boiled with 13 ml of ethyl acetate, cooled and filtered to give 0.73 g, m.p. 130—132.5°C.

Example 11

(dl)-N,N-Dichloro-4-[5-(hydroxymethyl)-2-oxooxazolidin-3-yl]benzenesulfonamide ($R_1$ = $NCl_2$, $R_5$ = H)

A 2 g portion of (*dl*)-4-[5-(hydroxymethyl)-2-oxooxazolidin-3-yl]benzenesulfonamide in a mixture of 10 ml water and 50 ml dichloromethane was stirred in an ice bath and 27 ml of 5.25% of sodium hypochlorite (Clorox) was added. The solid all dissolved and a precipitate formed. The filtered dichloromethane was separated, dried and evaporated to yield 1.54 g of the title compound, m.p. 121.5—123°C. A further 1.24 g was insoluble solid and had the same infrared curve as the above sample.

Example 12

(l)-N,N-Dichloro-4-[5-(hydroxymethyl)-2-oxooxazolidin-3-yl]benzenesulfonamide

A suspension of 1.00 g of (l)-4-[5-hydroxymethyl)-2-oxooxazolidin-3-yl]benzenesulfonamide in 1 ml water was stirred in an ice bath as 13.5 ml 5.25% sodium hypochlorite (Clorox) was added. The solution was then made slightly acidic by adding acetic acid; the product separated as white crystals (1.24 g), m.p. 124.5—125.5°C.

Example 13

(*dl*)-N-Chloro-4-[5-(hydroxymethyl)-2-oxooxazolidin-3-yl]benzenesulfonamide, sodium salt (R = NClNa, $R_5$ = H)

2.0 g of (*dl*)-4-[5-(Hydroxymethyl)-2-oxooxazolidin-3-yl]benzenesulfonamide was stirred in 10 ml water in an ice bath as 13.5 ml of 5.25% sodium hypochlorite (Clorox) was added. The solid dissolved and crystals then separated. This was filtered and washed with a small amount of cold water and dried. The yield of the title compound was 1.97 g (decomposes on heating above 138°C)

Example 14

(l)-N-Chloro-4-[5-(hydroxymethyl)-2-oxooxazolidin-3-yl]benzenesulfonamide, sodium salt

One gram of (l)-4-[5-(hydroxymethyl)-2-oxooxazolidin-3-yl]benzenesulfonamide in 1 ml of water was stirred in an ice bath as 6.5 ml sodium hypochlorite (Clorox, 5.25% sodium hypochlorite) was added. The solid dissolved, and then a white solid crystallized. The product was filtered and washed once with a small portion of ice water (1.15 g) m.p. 165—167°C (dec.).

Example 15

(*dl*)-N-{4-[5-(Hydroxymethyl)-2-oxooxazolidin-3-yl]phenylsulfonyl}-S,S-dimethylsulfilimine ($R_1$ = N = $S(CH_3)_2$, $R_5$ = H)

A suspension of 2.00 g (*dl*)-4-[5-(hydroxymethyl)-2-oxooxazolidin-3-yl]benzenesulfonamide in 10 ml water was stirred and 11.5 ml 5.25% sodium hypochlorite (Clorox) added. Almost all of the starting solid dissolved. The solution was filtered and 25 ml ethanol was added followed by 10 ml dimethyl sulfide. The mixture was stirred well for thirty minutes and then concentrated to a colorless glass. The product was dissolved in acetonitrile and chromatographed using a Waters Prep-500 HpLc. The yield of the pure title compound was 1.74 g, m.p. 162.5—164.5°C (a crystal transformation was observed at 136°C).

Example 16

(*dl*)-3-[4-Hydrazinosulfonyl)phenyl-5-(hydroxymethyl)-2-oxazolidin-2-one ($R_1$ = $NHNH_2$, $R_5$ = H)

A 30 g portion of (*dl*)-4-[5-(trifluoroacetoxymethyl)-2-oxooxazolidin-3-yl]benzenesulfonyl chloride was added to a solution of 6 ml hydrazine hydrate in 300 ml tetrahydrofuran kept at −10 to 0°C. After stirring for thirty minutes, the mixture was allowed to come to ambient temperature. The tetrahydrofuran was evaporated in a nitrogen stream, and the residue was diluted with water and filtered. The yield was 11.81 g, m.p. 172—174°C dec. The product was purified by dissolving it in 30 ml dimethyl sulfoxide, the solution filtered and diluted with methanol; yield 8.8 g, m.p. 173°C dec.

Example 17

(dl)-4-[5-(Acetoxymethyl)-2-oxooxazolidin-3-yl]benzenesulfonamide ($R_1$ = $NH_2$, $R_5$ = $OCH_3$)

A mixture of 19.3 g (0.10 mole) (dl)-5-(hydroxymethyl)-3-phenyl-2-oxazolidinone, 50 ml acetonitrile, 11 ml acetic anhydride and 0.1 g 4-dimethylaminopyridine was refluxed for 30 minutes. The mixture was concentrated under reduced pressure to yield a crude oil which IR and NMR confirmed to contain (dl)-5-(hydroxymethyl)-3-phenyl-2-oxooxazolidinone acetate.

The oil was added to 130 ml chlorosulfonic acid, stirred at ambient temperature for two hours and then poured onto three liters of ice. The oil which remained was extracted into methylene chloride and dried over sodium sulfate. The methylene chloride was concentrated and the remaining oil (10.9 g) was added to a mixture of 300 ml THF and 60 ml concentrated ammonium hydroxide cooled to −10°C to 0°C. THF was removed under reduced pressure, and the residue was diluted with water and filtered to yield 8.8 g, m.p. 196.5°—197°C. Recrystallization from acetonitrile yielded 6.3 g of the title compound, m.p. 199—200°C.

Example 18

l-5-Hydroxymethyl-3-phenyl-2-oxazolidinone

*Part A*

(R)-(+)-1-Benzyl glycerol (48 g or 0.263 mole) was prepared by the procedures shown in S. Takano, E. Goto, M. Hirama and Ogasawara in *Heterocycles, 16,* 381 (1981) and references cited therein. This was tosylated by the referenced procedure to give 57.8 g of a colorless oil.

A mixture of 37.1 g (0.15 mole) of N-phenyl-4-methylbenzenesulfonamide in 150 ml of dry DMF was stirred and 17 g of potassium *t*-butoxide was added followed by 55 g of the above tosylate. The mixture was heated at 95—100°C for 15 hours. The mixture was poured into ice-water and the product extracted into ether. The ether was back washed with water and dried over sodium sulfate to give 57.1 g of product. Thin layer analysis on silica gel showed three components and some starting sulfonanilide. The 57.1 g of product was dissolved in 1.5 l of ether, stirred with 100 ml of 25% aqueous sodium hydroxide, and filtered through a bed of Celite filter aid. The ether was concentrated to give 32.7 g of oil. This was chromatographed on silica gel columns eluting with 90% toluene: 10% ethyl acetate in two runs to give 17.02 g of solid product, m.p. 88—99°C. The product is 4-methyl-N-phenyl[2-hydroxy-3-(phenylmethoxy)propyl]benzenesulfonamide.

A solution of 16.5 g (0.049 mole) of 4-methyl-N-phenyl[2-hydroxy-3-(phenylmethoxy)propyl]benzenesulfonamide in 100 ml of dry 1,2-dimethoxyethane was stirred under nitrogen as a solution of sodium naphthalene radical anion (prepared by adding 20 ml of a 40% dispersion of sodium metal in mineral oil to a solution of 40 g of naphthalene in 100 ml of dry 1,2-dimethoxyethane while keeping the mixture 25—40°C under nitrogen) was added. Addition continued until the color of the mixture faded and was dark-green to black. At the end of the addition, the temperature was allowed to rise to 40°C. After stirring for thirty minutes, 20 ml of water was added followed by 125 ml of 20% sulfuric acid. The temperature was kept between 30—40°C during the acidification. The 1,2-dimethoxyethane was removed by vacuum distillation. The water and residue were extracted six times with toluene, two times with methylene chloride and were then sparged with nitrogen to remove the methylene chloride. The water was then saturated with sodium chloride and extracted five times with methylene chloride. The extracts were dried over anhydrous potassium carbonate, filtered and concentrated to give 6.0 g of colorless oil. This product is d-3-phenylamino-1,2-propanediol.

A 6.0 g (0.036 mole) portion of d-3-phenylamino-1,2-propanediol was combined with 10 ml of 1,2-dimethoxyethane and 5 ml of diethyl carbonate and refluxed. To the boiling solution was added 0.1 g sodium methoxide. After ten minutes a solid separated and 5 ml more 1,2-dimethoxyethane was added. Reflux was continued for thirty minutes, after which one ml of acetic acid was added and the mixture was concentrated under reduced pressure to leave 7.2 g of solid. This was extracted with hot acetonitrile, and the acetonitrile was concentrated to yield 1.8 g of the title compound, m.p. 136.5—138°C. The optical notation of the product $[\alpha]_D^{25}$ is −67.1° (C = 1 in acetonitrile).

*Part B*

A 1820 ml portion of aniline was heated with stirring under $N_2$ to 80—85°C. To this was added slowly 265 ml of freshly distilled glycidol at such as rate that the temperature was maintained at 85°—90°C. After the first 40—100 ml of glycidol was added, the external heat source was removed and the reaction temperature was thereafter controlled by rate of addition and by occasional cooling. After the addition was complete, heat was applied to keep the temperature at 80°—90° for two hours. The product, (dl)-3-phenylamino-1,3-propanediol, was distilled through a short column at 4.0—4.5 mm pressure to remove the aniline which distilled at 50—55°C (1.5 l recovered). The product was distilled at 0.05 mm, bp, 132—135°C; yield 588 g of a colorless viscous oil.

A solution of 1201 g (7.18 moles) of (dl)-3-phenylamino-1,3-propanediol in 1.9 l of chloroform was stirred and 600.1 g (3.95 mole) of l-mandelic acid was added. The mixture was heated to reflux to dissolve the solid. It was then allowed to cool slowly, the salt separating as crystals. After reaching room temperature, the solid was filtered and washed by stirring it with chloroform three times. The dried yield was 868 g of (d)-3-phenylamino-1,2-propanediol(l)-mandelate, m.p. 86—87°C. This was recrystallized by refluxing with 2.8 l chloroform, cooling slowly and filtering, and washing three times with chloroform, to give 754 g, m.p. 87—88°C.

A suspension of 845 g (d)-3-phenylamino-1,2-propanediol(l)-mandelate in 300 ml water was stirred in an ice-bath as a cold solution of 108 g sodium hydroxide in 300 ml water was added. The solution was saturated with sodium chloride and then extracted continuously with dichloromethane. The extract was concentrated under vacuum to yield 446.9 g of (d)-3-phenylamino-1,2-propanediol, a colorless oil. The optical notation of this product was [α]23 = +21° (C = 1 in ethanol, read in 200 cm tube).

A mixture of 441.5 g (2.60 mole) of (d)-3-phenylamino-1,2-propanediol, 325 ml of diethyl carbonate, 300 ml of 1,2-dimethoxyethane, 40 g potassium carbonate 1 g sodium methoxide was refluxed for two hours. At that time, 20 g more potassium carbonate was added. After 3.5 hours, a further 50 ml of diethyl carbonate was added.

The reflux was continued fifteen hours. A thin layer chromatogram showed that the reaction was complete. About one-half the solvent was removed under vacuum and 500 ml water and 40 ml acetic acid were added. The white solid was filtered and washed with water, 70% ethanol and ether yielding 401.7 g of the title compound, m.p. 137.5—138.5°C. This was crystallized from 1 l 95% ethanol to yield 368.1 g, m.p. 138.6—139.1°C. It was recrystallized from 700 ml of acetonitrile to give 330.4 g, m.p. 138.5—139.5°C. The optical rotation of the product $[\alpha]_D^{25}$ is −72.0 (C = 1 in acetonitrile)

Example 19

(dl)-4-[5-(Benzoyloxymethyl)-2-oxooxazolidin-3-yl]benzenesulfonamide ($R_1$ = $NH_2$, $R_5$ = $OC_6H_5$)

A solution of 5.0 g (18.3 mmole) of (dl)-4-[5-(hydroxymethyl)-2-oxooxazolidin-3-yl]benzenesulfonamide in 50 ml of dry pyridine was stirred with cooling at −8°C to 0°C as 2.34 ml of benzoyl chloride was slowly added. A small exotherm was noted. After the addition was complete, the mixture was allowed to warm to 25°C, and stirring was continued for two hours. A further 0.2 ml of benzoyl chloride was added and the mixture was allowed to stand overnight. The mixture was then poured on ice and the product crystallized. It was filtered and washed well with water and dried. The yield was 6.72 g. This was recrystallized by dissolving in hot diethylene glycol dimethyl ether, concentrating to one half volume and letting stand; yield 3.55 g, m.p. 233—234°C.

Example 20

(dl)-4-[5-(Propionyloxymethyl)-2-oxooxazolidin-3-yl]benzenesulfonamide ($R_1$ = $NH_2$, $R_5$ = $OC_3H_7$)

A solution of 5.0 g (18.3 mmole) of (dl)-4-[5-(hydroxymethyl)-2-oxooxazolidin-3-yl]benzenesulfonamide in 50 ml of dry pyridine was stirred in an ice bath as 1.60 ml of propionyl chloride was added. The mixture was allowed to warm to ambient temperature and stand overnight. It was then poured into 200 ml of ice-water, the water made acid with hydrochloric acid while being kept cold, and the crystalline product was filtered and washed with cold water. The yield was 3.60 g, m.p. 211—212.5°C. This was recrystallized from nitromethane to give 3.32 g, m.p. 212—213.5°C.

Example 21

(dl)-Butanedioic acid, Mono Ester with 4-[5-(Hydroxymethyl)-2-oxooxazolidin-3-yl]benzenesulfonamide ($R_1$ = $NH_2$, $R_5$ = $C(O)CH_2CH_2COOH$)

A solution of 5.0 g (18.3 mmole) of (dl)-4-[5-(hydroxymethyl)-2-oxooxazolidin-3-yl]benzenesulfonamide in 50 ml of dry pyridine and 2.02 g of succinic anhydride was stirred and heated for two hours at 60°C. The reaction mixture was poured into 100 ml of ice-water and the pH was brought to 3 by adding concentrated hydrochloric acid (while keeping cool). The solution was saturated with sodium chloride and extracted with tetrahydrofuran. The extract was dried over sodium sulfate and concentrated to give 7.96 g of crystals. The solid was stirred in 50 ml of water, and 20% potassium bicarbonate was added until the pH = 10. The product was then filtered and the filtrate was brought back to pH = 2 with hydorchloric acid. The product was filtered and dried to yield 4.80 g, m.p. 167—170°C.

The sodium salt of this product was prepared by suspending 2.4 g of the above solid in 50 ml of water, adding 1N NaOH until the pH was 7, filtering and concentrating under vacuum to give 2.35 g of white solid.

In like manner, the following half esters may be prepared.

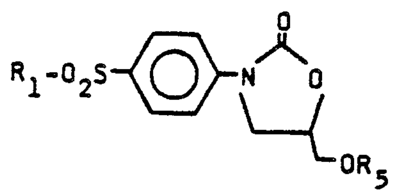

(I)

| Ex. | Starting Compound $R_1$ | $R_5$ | Reactant | Product $R_1$ | Product $R_5$ |
|---|---|---|---|---|---|
| 22 | $H_2N-$ | H | 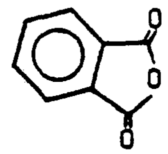 | $H_2N-$ | 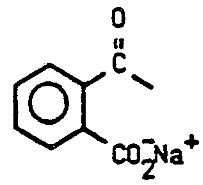 |
| 23 | $Cl_2N-$ | H | 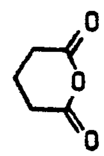 | $Cl_2N-$ | 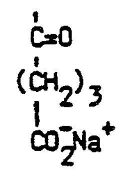 |
| 24 | $N_3-$ | H | 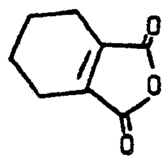 | $H_2N-$ | 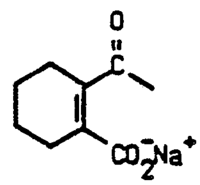 |
| 25 | $CH_3NH-$ | H | 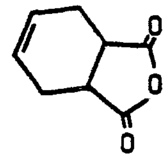 | $CH_3NH-$ | 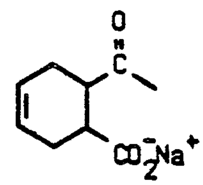 |
| 26 | $(CH_3)_2S{=}N-$ | H | 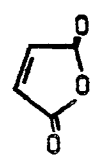 | $(CH_3)_2S{=}N-$ | 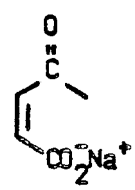 |
| 27 | $CH_3OHN-$ | H | 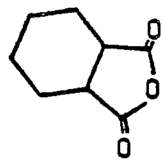 | $CH_3OHN-$ | 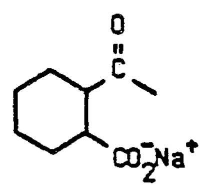 |

### Example 28

L-Alanine Ester with *l*-[5-(Hydroxymethyl)-2-oxooxazolidin-3-yl]benzenesulfonamide ($R_1 = NH_2$, $R_5 = COCH(NH_2)CH_3$)

The title compound can be made as follows.

A solution of 4.5 g (0.02 mole) of N,N-dicyclohexylcarbodiimide in methylene chloride is added to a solution of 5.4 g (0.02 mole) of l-[5-(hydroxymethyl)-2-oxooxazolidin-3-yl]benzenesulfonamide and 3.8 g (0.02 mole) of N-*t*-butoxycarbonyl-L-alanine, 1.2 g of pyridine and 100 ml of dichloromethane. The mixture is stirred at ambient temperature for one day and the solid precipitate filtered out. The filtrate is washed successively with 1M potassium bisulfate, water, and dilute sodium bicarbonate and then dried over

sodium sulfate. The solvent is concentrated and the residue recrystallized from a suitable solvent.

The solid is dissolved in trifluoroacetic acid at 0°C, stirred for fifteen minutes and poured into ether. A solid precipitate forms, and is filtered off and washed with ether. This is the trifluoroacetate of the desired L-alanyl ester.

Using the procedure described above, the following compounds may be prepared.

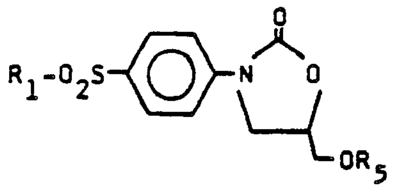

(I)

| | Starting Compound | | N-*t*-BOC Amino Acid | Product | |
|---|---|---|---|---|---|
| Ex. | $R_1$ | $R_5$ | | $R_1$ | $R_5$ |
| 29 | $CH_3HN$— | H | Ph-$CH_2CHCO_2H$<br>\|<br>*t*-BOC—NH | $CH_3HN$— | O<br>‖<br>—CCH—$CH_2$-Ph<br>\|<br>$NH_2$ |
| 30 | $(Cl)_2N$— | H | *t*-BOCNH$CH_2CO_2H$ | $(Cl)_2N$— | O<br>‖<br>—C$CH_2$<br>\|<br>$NH_2$ |
| 31 | $N_3$— | H | $(CH_3)_2CHCHCO_2H$<br>\|<br>*t*-BOC—NH | $N_3$— | O<br>‖<br>—C$CH_2$—$CH(CH_3)_2$<br>\|<br>$NH_2$ |
| 32 | $C_2H_5HN$— | H | $(CH_3)_2CHCH_2CHCO_2H$<br>\|<br>*t*-BOC—NH | $C_2H_5HN$— | O<br>‖<br>—C$CH_2CH_2CH(CH_3)_2$<br>\|<br>$NH_2$ |
| 33 | $(CH_3)_2S$=N— | H | Ph-$CH_2CHCO_2H$<br>\|<br>*t*-BOC—NH | $(CH_3)_2S$=N— | O<br>‖<br>—CCH—$CH_2$-Ph<br>\|<br>$NH_2$ |
| 34 | $CH_3OHN$— | H | *t*-BOCNH$CH_2CO_2H$ | $CH_3OHN$— | O<br>‖<br>—C$CH_2$<br>\|<br>$NH_2$ |

Dosage Forms

The antibacterial agents of this invention can be used for the manufacture of medicaments which are administered by any means that produces contact of the active agent with the agent's site of action in the body of a mammal. The medicaments can be administered by any conventional means available for use in conjunction with pharmaceuticals; either as individual therapeutic agents or in a combination of therapeutic agents. They can be administered alone, but are generally administered with a pharmaceutical carrier selected on the basis of the chosen route of administration and standard pharmaceutical practice.

The dosage administered will, of course, vary depending upon known factors such as the pharmacodynamic characteristics of the particular agent, and its mode and route of administration; age, health and weight of the recipient; nature and extent of symptoms, kind of concurrent treatment, frequency of treatment, and the effect desired. Usually a daily dosage of active ingredient in the medicament administered can be about 5 to 20 milligrams per kilogram of body weight. Ordinarily, when the more

potent compounds of this invention are used, 5 to 15, and preferably 5 to 7.5 milligrams per kilogram per day, given in divided doses 2 to 4 times a day or in sustained release form, is effective to obtain desired results. The medicaments containing these drugs may also be administered parenterally.

Dosage forms (compositions) manufactured for internal administration contain from about 1.0 milligram to about 500 milligrams of active ingredient per unit. In these pharmaceutical compositions the active ingredient will ordinarily be present in an amount of about 0—95% by weight based on the total weight of the composition.

The medicaments containing the active ingredient can be administered orally in solid dosage forms, such as capsules, tablets, and powders, or in liquid dosage forms, such as elixirs, syrups, and suspensions, they can also be administered parenterally, in sterile liquid dosage forms.

Gelatin capsules contain the active ingredient and powdered carriers, such as lactose, sucrose, mannitol, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration contain preferably a water soluble salt of the active ingredient, suitable stabilizing agents, and if necessary, buffer substances. Antioxidizing agents such as sodium bisulfate, sodium sulfite, or ascorbic acid either alone or combined are suitable stabilizing agents. Also used are citric acid and its salts and sodium EDTA. In addition parenteral solutions can contain preservatives, such as benzalkonium chloride, methyl- or propyl-paraben, and chlorobutanol.

Suitable pharmaceutical carriers are described in *Remington's Pharmaceutical Sciences,* E. W. Martin, a standard reference text in this field.

Useful pharmaceutical dosage forms prepared in the manufacture of a medicament for administration of the compounds of this invention can be illustrated as follows:

Capsules

A large number of unit capsules are prepared by filling standard two-piece hard gelatin capsules each with 75 milligrams of powdered active ingredient, 150 milligrams of lactose, 24 milligrams of talc and 6 milligrams magnesium stearate.

A mixture of active ingredient in soybean oil is prepared and injected by means of a positive displacement pump into gelatin to form soft gelatin capsules containing 75 milligrams of the active ingredient. The capsules are washed in petroleum ether and dried.

Tablets

A large number of tablets are prepared by conventional procedures so that the dosage unit is 75 milligrams of active ingredient, 0,2 milligrams of colloidal silicon dioxide, 5 milligrams of magnesium stearate, 250 milligrams of microcrystalline cellulose, 11 milligrams of cornstarch and 98.8 milligrams of lactose. Appropriate coatings may be applied to increase palatability or delay absorption.

Injectable

A parenteral composition suitable for administration by injection is prepared by stirring 1.5% by weight of active ingredient in 10% by volume propylene glycol and water. The solution is made isotonic with sodium chloride and sterilized.

Suspension

An aqueous suspension is prepared for oral administration so that each 5 milliliters contain 75 milligrams of finely divided active ingredient, 200 milligrams of sodium carboxymethyl cellulose, 5 milligrams of sodium benzoate, 1.0 grams of sorbitol solution, U.S.P., and 0.025 milliliters of vanillin.

Utility

Test results indicate that the novel compounds of this invention are active against a wide variety of aerobic, facultative anaerobic and obligate anaerobic bacterial isolates including beta-lactamase producing *Staphylococcus aureus* and *Neisseria gonorrhea* strains, members of the gram-negative *Enterobacteriaceae* and *Pseudomonas aeruginosa*. The antibacterial spectrum of these agents encompasses organisms recognized as major human and veterinary pathogens of the respiratory, gastrointestinal, genito-urinary and central nervous systems; blood; interstitial fluids, soft tissue; and bone.

A list is presented in Table I of analogs which exert an *in-vitro* antibacterial effect. A standard microdilution method (Conrath, Theodore B., *1972 Handbook of Microtiter Procedures,* Dynatech Corporation, Cambridge, Massachusetts) with Mueller-Hinton broth was used to determine the 24 hour minimal inhibitory concentrations (MIC's) for test strains of *Staphylococcus epidermidis* and *E. Coli.*

TABLE I
*In Vitro* Broth Dilution Minimal Inhibitory Concentrations

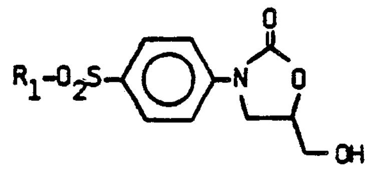

| $R_1$ | Chemical Name | Microdilution Broth MIC: µg/ml S. epidermidis | E. coli |
|---|---|---|---|
| $NH_2$ | (d,l)-4-[5-(hydroxymethyl)-2-oxooxazolidin-3-yl]benzene-sulfonamide | 62.5 | 62.5 |
| $NH_2$ | (l)-4-[5-(hydroxymethyl)-2-oxooxazolidin-3-yl]benzene-sulfonamide | 31.3 | 31.3 |
| $N(CH_3)_2$ | (d,l)-4-[5-(hydroxymethyl)-2-oxo-oxazolidin-3-yl]-N,N-dimethyl-benzenesulfonamide | 50.0 | >200.0 |
| $CH_3NH$ | (d,l)-4-[5-(hydroxymethyl)-2-oxo-oxazolidin-3-yl]-N-methyl-benzenesulfonamide | 50.0 | >200.0 |
| $CH_3NH$ | (l)-4-[5-(hydroxymethyl)-2-oxo-oxazolidin-3-yl]-N-methyl-benzenesulfonamide | 25.0 | 200.0 |
| $N_3$ | (d,l)-4-[5-(hydroxymethyl)-2-oxooxazolidin-3-yl]benzene-sulfonyl azide | 4.2 | 12.5 |
| $N_3$ | (l)-4-[5-(hydroxymethyl)-2-oxooxazolidin-3-yl]benzene-sulfonyl azide | 1.6 | 9.4 |

A summary of the *in-vitro* antibacterial spectrum of (l)-4-[5-(hydroxymethyl)-2-oxooxazolidin-3-yl]benzenesulfonamide, is shown in Table II. The minimal inhibitory concentrations for the aerobic and facultative anaerobic test isolates representing two gram-positive and eleven gram-negative general were determined by an agar dilution method. The method is state of the art and is as follows. Mueller-Hinton agar plates are prepared containing two-fold compound concentrations ranging from 128 µg/ml to 1.0 g/ml for the suspectibility testing of the bacterial strains except *Neisseria gonorrhoea* and *Hemophilus sp.*. The latter organisms are tested using GC agar containing 1% Bacto-Supplement C (Difco Laboratories, Detroit, Michigan) and are incubated under 6% $CO_2$. The agar plates are inoculated with a 0.001 ml calibrated loopful of bacterial inoculum diluted to contain $5 \times 10^6$ colony-forming units (CFU) per ml. After a 24-hour incubation period at 35°C the MIC's are recorded as the lowest compound concentration which inhibits macroscopic bacterial growth.

The agar dilution method described by A. L. Barry ("The Antimicrobic Susceptibility Test: Principles and Practice", 1976, Lea and Febiger, Philadelphia, Pennsylvania) with compound concentrations of 32 µg/ml to 0.06 µg/ml, was used to determine the inhibitory activity of (l)-4-[5-(hydroxymethyl)-2-oxooxazolidin-3-yl]benzenesulfonamide for anaerobic bacteria.

TABLE II
*In Vitro* Antibacterial Spectrum of
(l)-4-[5-(Hydroxymethyl)-2-oxooxazolidin-3-yl]benzenesulfonamide

| Test Organism | No. Isolates | Mean MIC[1]: μg/ml |
|---|---|---|
| *Staphylococci* sp. | 38 | 22.3 |
| *Streptococci* sp. | 14 | 7.7 |
| *E. coli* | 86 | 29.8 |
| *Proteus sp.* | 88 | 29.1 |
| *Providencia sp.* | 12 | 41.3 |
| *Enterobacter sp.* | 41 | 58.5 |
| *Salmonella sp.* | 6 | 32 |
| *Shigella sp.* | 9 | 8.0 |
| *Serratia sp.* | 44 | ⩾96.0 (Range 32→128) |
| *Klebsiella sp.* | 50 | 39.4 |
| *Pseudomonas sp.* | 79 | ⩾116.8 (Range 8→128) |
| *Neisseria sp.* | 43 | 12.3 |
| *Haemophilus sp.* | 4 | 32 |
| *Clostridium sp.* | 5 | 16.8 |
| *Fusobacterium sp.* | 4 | 0.9 |
| *Bacteroides sp.* | 23 | ⩾15.13 (Range 4→32) |
| Gram — Anaerobic Cocci | 7 | 6.1 |

[1]MIC: Minimal Inhibitory Concentrations by agar dilution method.

The *in-vivo* potency of these compounds is exemplified by the data summarized in Table III. *In-vivo* efficacy determinations were performed by inoculating mice intraperitoneally with cultures of the infecting organism diluted to produce 90—100% mortality in control animals within seven days. The diluents used were trypticase soy broth for *E. coli, Proteus sp,* and *Pseudomonas aeruginosa* and 5% hog gastric mucin for *Staphylococcus aureus* infections. The compounds, dissolved or suspended in 0.25% methocel·$H_2O$ were used to prepare medicaments, which were administered orally by intubation at the time of infection and again at four hours post-infection. Mortality was recorded daily until test termination and the 50 percent effective dose, $ED_{50}$, was calculated by the Reed-Muench method (Reed, L. G. and Muench, H., "A simple method of estimating fifty percent end points". *American Journal of Hygiene, 27,* 1938, 493—497).

TABLE III

*In Vivo* Efficacy or Orally Administered Compounds in Mouse Intraperitoneal Infections

*Chemical 1*
(d,l)-4-[5-(hydroxymethyl)-2-oxooxazolidin-3-yl]benzenesulfonamide
*Chemical 2*
(l)-4-[5-(hydroxymethyl)-2-oxooxazolidin-3-yl]benzenesulfonamide
*Chemical 3*
(d,l)-4-[5-(hydroxymethyl)-2-oxooxazolidin-3-yl]benzenesulfonyl azide
*Chemical 4*
(l)-4-[5-(hydroxymethyl)-2-oxooxazolidin-3-yl]-N-methylbenzenesulfonyl azide
*Chemical 5*
(l)-4-[5-(hydroxymethyl)-2-oxooxazolidin-3-yl]-N-methylbenzenesulfonamide

| | | Infecting Bacterial Organism | | | | |
|---|---|---|---|---|---|---|
| $R_1$ | Chem. | Staphylococcus aureus $ED_{50}$[1] | Escherichia coli $ED_{50}$ | Proteus mirabilis $ED_{50}$ | Proteus vulgaris $ED_{50}$ | Pseudomonas fluorescens $ED_{50}$ |
| $NH_2$ | 1 | 39.1 | 24.9 | N.T.[2] | N.T. | N.T. |
| $NH_2$ | 2 | 22.4 | 13.9 | 44.3 | 40.8 | 67.46 |
| $N_3$ | 3 | 25.99 | 17.89 | N.T. | N.T. | N.T. |
| $N_3$ | 4 | 26.4 | 14.87 | N.T. | N.T. | N.T. |
| $CH_3NH$ | 5 | 15.44 | 39.72 | N.T. | N.T. | N.T. |

[1]$ED_{50}$: 50 percent effective dose: mg/kg
[2]N.T.: Not tested.

**Claims**

1. A compound of the formula

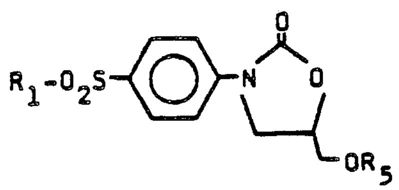

(I)

where
$R_1$ is $-NR_2R_3$, $-N(OR_2)R_3$, $-N_3$, $-NHNH_2$, $-NX_2$, $-NR_{10}X$, $-NXZ$ or $-N{=}S(O)_nR_8R_9$;
$R_2$ and $R_3$ are independently H, alkyl of 1—4 carbons or cycloalkyl of 3—8 carbons;
$R_5$ is

$R_5$ is H, $-\overset{O}{\overset{\|}{C}}R_6$, $-\overset{O}{\overset{\|}{C}}(CH_2)_m\overset{O}{\overset{\|}{C}}-OH$, $-\overset{O}{\overset{\|}{C}}-CH{=}CH-\overset{O}{\overset{\|}{C}}-OH$,

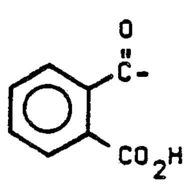 , 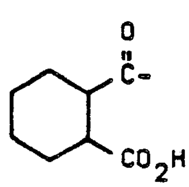 , 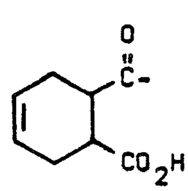 ,

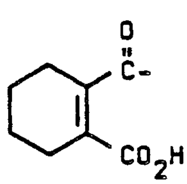 or $-\overset{O}{\overset{\|}{C}}-\underset{NH_2}{\underset{|}{CH}}-R_7$ ;

$R_6$ is aryl or alkyl of 1—12 carbons;
$R_7$ is H, alkyl of 1—5 carbons, —$CH_2OH$, $CH_2SH$, aryl or aralkyl;
$R_8$ and $R_9$ are independently alkyl of 1—4 carbons or, taken together are —$(CH_2)_p$—;
$R_{10}$is alkyl of 1—4 carbons;
X is Cl or Br;
Z is a physiologically acceptable cation;
m is 2 or 3;
n is 0 or 1; and
p is 3, 4 or 5;

2. A compound of Claim 1 where $R_5$ = H.

3. A compound of Claim 1 where $R_1 = NH_2$.

4. The compound of Claim 1 which is (l)-4-[5-(hydroxymethyl)-2-oxooxazolidin-3-yl]benzenesulfonamide.

5. A pharmaceutical composition consisting essentially of a suitable pharmaceutical carrier and an effective antibacterial amount of a compound of Claim 1.

6. A pharmaceutical composition consisting essentially of a suitable pharmaceutical carrier and an effective antibacterial amount of a compound of any of Claims 2 to 4.

7. Use of an effective antibacterial amount of a compound of Claim 1 for the manufacture of a medicament for alleviating bacterial infection in a mammal.

8. Use of an effective antibacterial amount of a compound of any of Claims 2 to 4 for the manufacture of a medicament for alleviating bacterial infection in a mammal.

9. A process for preparing compounds of Claim 1 comprising, in a manner known per se,

(a) contacting a compound of the formula

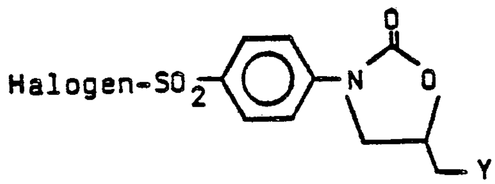

where Y is a substituent containing an acyl group, with a reactant selected from an appropriate amine $HNR_2R_3$ or $HN(OR_2)R_3$, azide ion $^-N_3$, or hydrazine, to prepare a compound of the formula

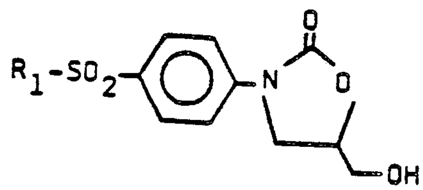

where

$R_1 = NR_2R_3$, $N(OR_2)R_3$,$N_3$ or $NHNH_2$;

(b) optionally contacting the product of step (a) where $R_1 = NH_2$ with a preformed hypohalite or with halogen and a base to prepare a compound of the formula

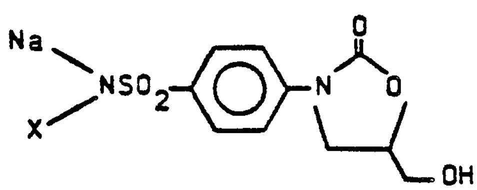

where

X = Cl or Br;

(c) optionally contacting the product of step (b) with

(i) additional hypohalite at a pH of about 4 to 8 to prepare a compound of the formula

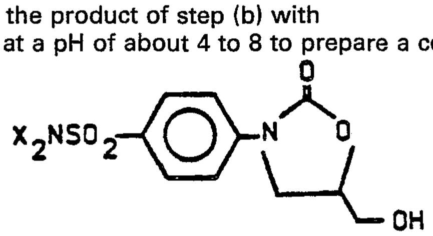

where

X = Cl or Br; or

(ii) a dialkyl sulfide $SR_8R_9$, to prepare a compound of the formula

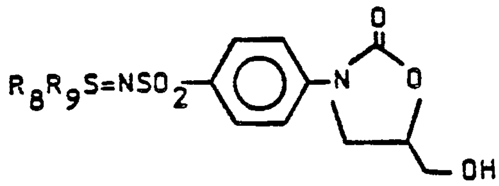 ;

(d) optionally oxidizing the product of step (c) (ii) to prepare a compound of the formula

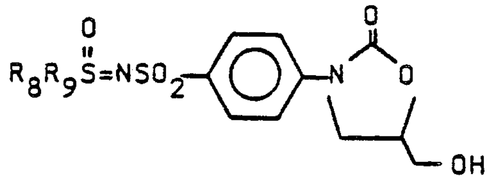 ;

(e) optionally contacting the product of step (a) where $R_1 = NH_2R_3$, $R_2 = H$ and $R_3$ = alkyl of 1—4 carbons, with hypohalite to prepare the compound

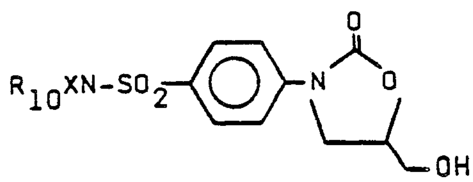 ;

(f) optionally contacting the product of any of steps (a)—(e) with
(i) an acid chloride $R_6COCl$ or anhydride $(R_6CO)_2O$ to prepare a compound of the formula

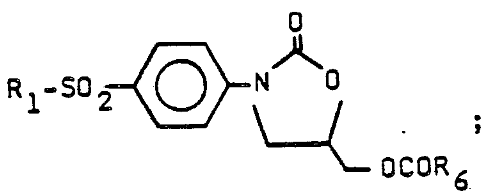 ;

(ii) with an anhydride of the formula

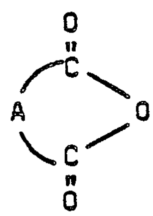

where
A = —CH=CH—, $-(CH_2)_m-$,

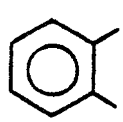 , 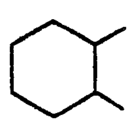 , 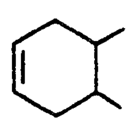 or 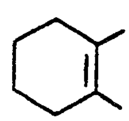 ,

and m = 2 or 3, to prepare a compound of the formula

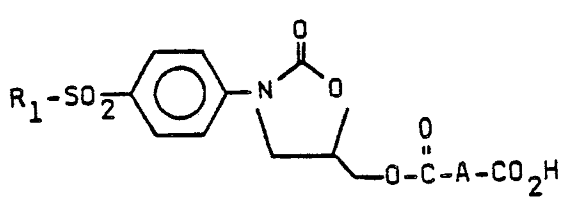 ;

(iii) a blocked amino acid of the formula

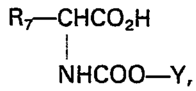

where Y is a protective group such as *tert*-butyl, in the presence of a carbodiimide followed by a volatile acid, HX, to prepare a compound of the formula

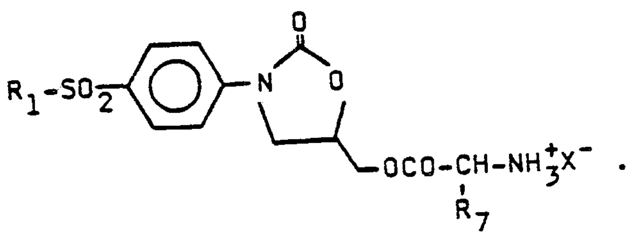

10. A process for resolving 3-phenylamino-1,2-propanediol comprising — in a manner known per se — contacting racemic 3-phenylamino-1,2-propanediol with about 50 to 60 mole percent of optically active mandelic acid to obtain a salt and contacting said salt with base to provide 3-phenylamino-1,2-propanediol enriched in one enantiomer.

11. The process of Claim 10 where the optically active mandelic acid is l-mandelic acid and the product 3-phenylamino-1,2-propanediol is enriched in the dextrorotatory (R) enantiomer.

**Patentansprüche**

1. Verbindung der Formel

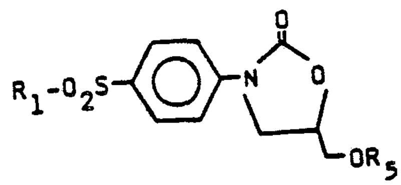

(I)

in der

$R_1$ —$NR_2R_3$, —$N(OR_2)R_3$, —$N_3$, —$NHNH_2$, —$NX_2$, —$NR_{10}X$, —NXZ oder —$N=S(O)_nR_8R_9$ ist,

$R_2$ und $R_3$ unabhängig voneinander H, Alkyl mit 1 bis 4 Kohlenstoff-Atomen oder Cycloalkyl mit 3 bis 8 Kohlenstoff-Atomen ist,

$R_5$

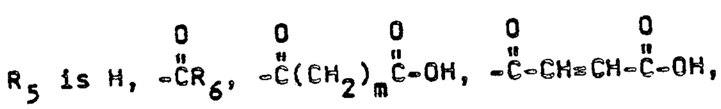

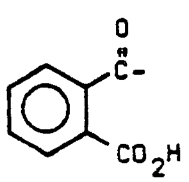 , 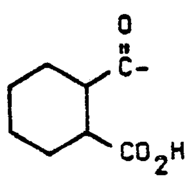 , 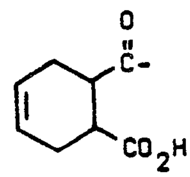 ,

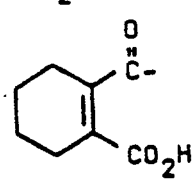 oder 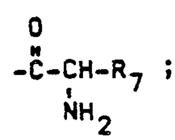 ;

$R_6$ Aryl oder Alkyl mit 1 bis 12 Kohlenstoff-Atomen ist

$R_7$ H, Alkyl mit 1 bis 5 Kohlenstoff-Atomen, —$CH_2OH$, $CH_2SH$, Aryl oder Aralkyl ist,

$R_8$ und $R_9$ unabhängig voneinander H, Alkyl mit 1 bis 4 Kohlenstoff-Atomen oder, zusammen genommen, —$(CH_2)_p$— sind,

$R_{10}$ Alkyl mit 1 bis 4 Kohlenstoff-Atomen ist,

X Cl oder Br ist,

Z ein physiologisch unbedenkliches Kation ist,
m 2 oder 3 ist,
n 0 oder 1 ist und
p 3, 4 oder 5 ist.

2. Verbindung nach Anspruch 1, dadurch gekennzeichnet, daß $R_5$ H ist.

3. Verbindung nach Anspruch 1, dadurch gekennzeichnet, daß $R_1$ $NH_2$ ist.

4. Verbindung nach Anspruch 1, die (l)-4-[5-(Hydroxymethyl)-2-oxooxazolidin-3-yl]benzolsulfonamid ist.

5. Pharmazeutische Zusammensetzung, bestehend im wesentlichen aus einem geeigneten pharmazeutischen Träger und einer wirksamen antibakteriellen Menge einer Verbindung nach Anspruch 1.

6. Pharmazeutische Zusammensetzung, bestehend im wesentlichen aus einem geeigneten pharmazeutischen Träger und einer wirksamen antibakteriellen Menge einer Verbindung nach irgendeinem der Ansprüche 2 bis 4.

7. Verwendung einer wirksamen antibakteriellen Menge einer Verbindung nach Anspruch 1 zur Herstellung eines Medikaments zur Linderung bakterieller Infektionen bei Säugern.

8. Verwendung einer wirksamen antibakteriellen Menge einer Verbindung nach irgendeinem der Ansprüche 2 bis 4 zur Herstellung eines Medikaments zur Linderung bakterieller Infektionen bei Säugern.

9. Verfahren zur Herstellung von Verbindungen nach Ansprüch 1, dadurch gekennzeichnet, daß in an sich bekannter Weise

(a) eine Verbindung der Formel

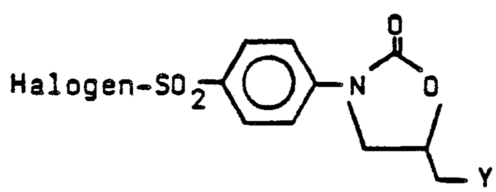

in der

Y ein eine Acyl-Gruppe enthaltender Substituent ist, mit einem aus einem geeigneten Amin $HNR_2R_3$ oder $HN(OR_2)R_3$, Azid-Ion $^-N_3$ oder Hydrazin in Berührung gebracht wird, um eine Verbindung der Formel

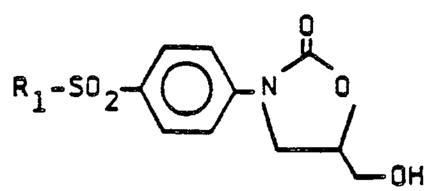

herzustellen, in der

$R_1$ $NR_2R_3$, $N(OR_2)R_3$, $N_3$ oder $NHNH_2$ ist;

(b) gegebenenfalls das Produkt aus Schritt (a), in dem $R_1$ $NH_2$ ist, mit einem vorher gebildeten Hypohalogenit oder mit Halogen und einer Base in Berührung gebracht wird, um eine Verbindung der Formel

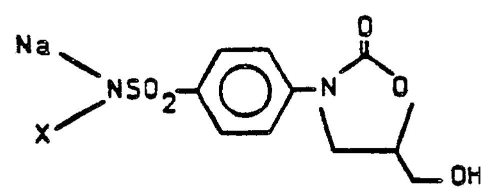

herzustellen, in der

X Cl oder Br ist;

(c) gegebenenfalls das Produkt aus Schritt (b)

(i) mit zusätzlichem Hypohalogenit bei einem pH von 4 bis 8 in Berührung gebracht wird, um eine Verbindung der Formel

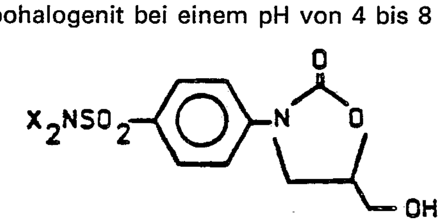

herzustellen, in der

X Cl oder Br ist, oder

(ii) mit einem Dialkylsulfid $SR_8R_9$ in Berührung gebracht wird, um eine Verbindung der Formel

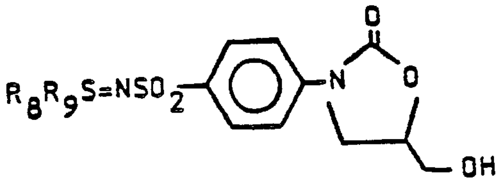 ;

herzustellen;

(d) gegebenenfalls das Produkt aus Schritt (c) (ii) oxidiert wird, um eine Verbindung der Formel

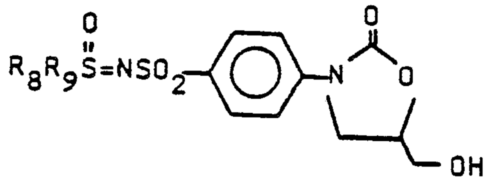 ;

herzustellen;

(e) gegebenenfalls das Produkt aus Schritt (a), in dem $R_1 = NR_2R_3$, $R_2 = H$ und $R_3$ = Alkyl mit 1 bis 4 Kohlenstoff-Atomen, mit Hypohalogenit in Berührung gebracht wird, um eine Verbindung der Formel

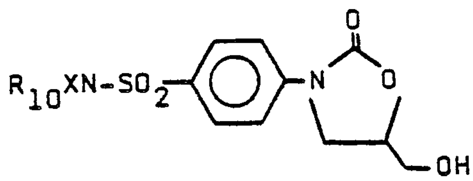 ;

herzustellen;

(f) gegebenenfalls das Produkt aus irgendeinem der Schritte (a) bis (e) mit

(i) einem Säurechlorid $R_6COCl$ oder -anhydrid $(R_6CO)_2O$ in Berührung gebracht wird, um eine Verbindung der Formel

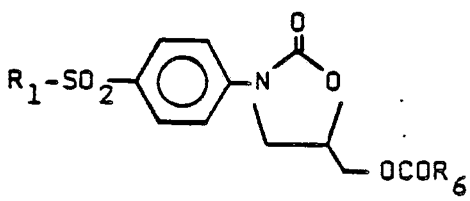 ;

herzustellen;

(ii) mit einem Anhydrid der Formel

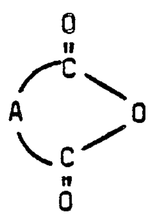

in der A = —CH=CH—, $-(CH_2)_m-$,

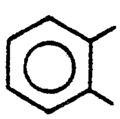 , 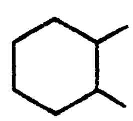 , 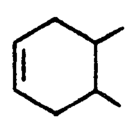 oder 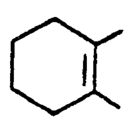 ,

und m = 2 oder 3, in Berührung gebracht wird, um eine Verbindung der Formel

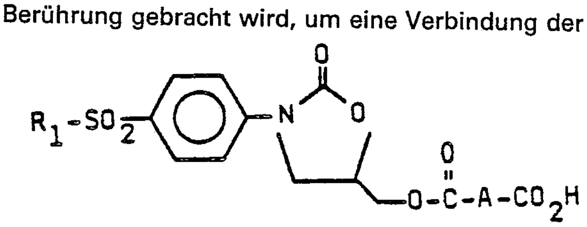 ;

herzustellen;

(iii) mit einer blockierten Aminosäure der Formel

$R_7$—CHCO$_2$H
|
NHCOO—Y,

in der Y eine Schutzgruppe wie tert-Butyl ist, in Gegenwart eines Carbodiimids, gefolgt von einer flüchtigen Säure HX, in Berührung gebracht wird, um eine Verbindung der Formel

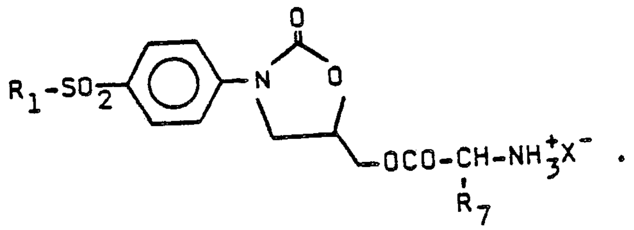

herzustellen.

10. Verfahren zur Auflösung von 3-Phenylamino-1,2-propandiol, dadurch gekennzeichnet, daß — in an sich bekannter Weise — das racemische 3-Phenylamino-1,2-propandiol mit etwa 50 bis 60 Mol-% optisch aktiver Mandelsäure in Berührung gebracht wird, um ein Salz zu gewinnen, un dieses Salz mit einer Base in Berührung gebracht wird, um 3-Phenylamino-1,2-propandiol zu gewinnen, in dem ein Enantiomer angereichert ist.

11. Verfahren nach Anspruch 10, dadurch gekennzeichnet, daß die optisch aktive Mandelsäure l-Mandelsäure ist, und das Produkt 3-Phenylamino-1,2-propandiol mit dem rechtsdrehenden (R)-Enaniomer angereichert ist.

**Revendications**

1. Un composé de la formule:

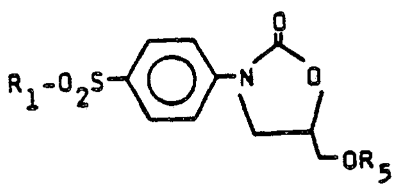

où

$R_1$ est —$NR_2R_3$, —$N(OR_2)R_3$, —$N_3$, —$NHNH_2$, —$NX_2$, —$NR_{10}X$, —NXZ ou —$N{=}S(O)_nR_8R_9$;

$R_2$ et $R_3$ sont indépendamment H, un groupe alcoyle de 1—4 atomes de carbone ou cycloalcoyle de 3—8 atomes de carbone;

$R_5$ est

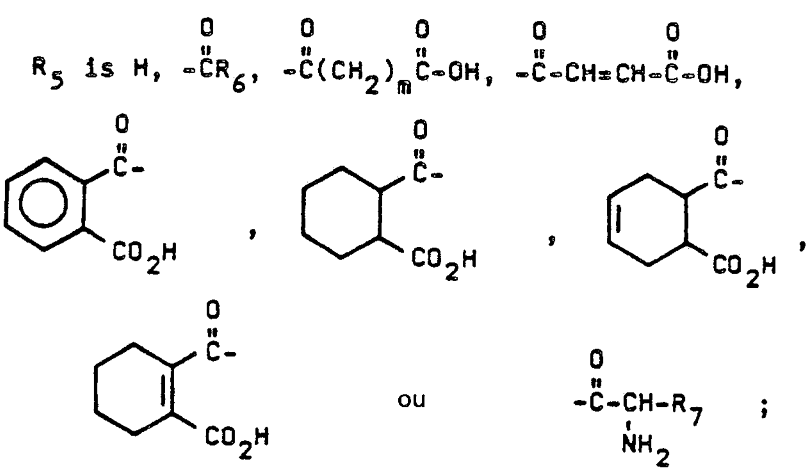

ou

$R_6$ est un groupe aryle ou alcoyle de 1—12 atomes de carbone;

$R_7$ est H, un groupe alcoyle de 1—5 atomes de carbone, —$CH_2OH$, $CH_2SH$, aryle ou aralcoyle;

$R_8$ et $R_9$ sont indépendamment des groupes alcoyle de 1—4 atomes de carbone ou, pris en même temps, sont —$(CH_2)_p$—;

$R_{10}$ est un groupe alcoyle de 1—4 atomes de carbone;

X est Cl ou Br;

Z est un cation physiologiquement acceptable;
m est 2 ou 3;
n est 0 ou 1; et
p est 3, 4 ou 5.

2. Un composé selon la revendication 1, dans lequel $R_5 = H$.

3. Un composé selon la revendication 1, dans lequel $R_1 = NH_2$.

4. Le composé selon la revendication 1, qui est le (l)-4-[5-(hydroxyméthyl)-2-oxo-oxazolidin-3-yl]benzènesulfonamide.

5. Une composition pharmaceutique constituée essentiellement d'un véhicule pharmaceutique approprié et d'une quantité anti-bactérienne efficace d'un composé selon la revendication 1.

6. Une composition pharmaceutique constituée essentiellement d'un véhicule pharmaceutique approprié et d'une quantité antibactérienne efficace d'un composé selon l'une quelconque des revendications 2 à 4.

7. L'utilisation d'une quantité antibactérienne efficace d'un composé selon la revendication 1 pour la préparation d'un médicament destiné à lutter contre une infection bactérienne chez un mammifère.

8. L'utilisation d'une quantité antibactérienne efficace d'un composé selon l'une quelconque des revendications 2 à 4 pour la préparation d'un médicament destiné à lutter contre une infection bactérienne chez un mammifère.

9. Un procédé pour la préparation de composés de la revendication 1 comprenant, d'une manière en elle-même connue

(a) la mise en contact d'un composé de la formule

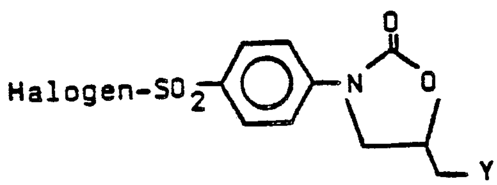

ou Y est un substituant contenant un groupe acyle, avec un corps en réaction choisi parmi une amine appropriée $HNR_2R_3$ ou $HN(OR_2)R_3$, l'ion azoture $^-N_3$, ou l'hydrazine, afin de préparer un composé de la formule

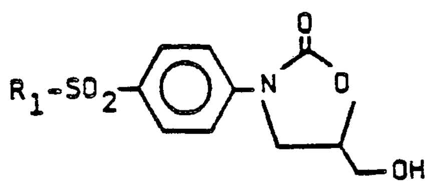

où

$R_1 = NR_2R_3$, $N(OR_2)R_3$, $N_3$ ou $NHNH_2$;

(b) facultativement la mise en contact du produit de l'étape (a) dans lequel $R_1 = NH_2$ avec un hypohalogénite formé à l'avance ou avec un halogène et une base de façon à préparer un composé de la formule

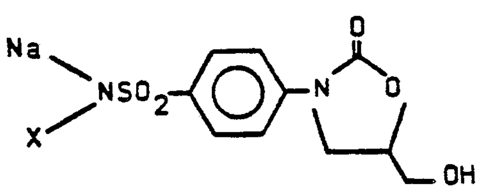

où

X = Cl ou Br;

(c) facultativement la mise en contact du produit de l'étape (b) avec

(i) une quantité supplémentaire d'hypohalogénite à un pH d'environ 4 à 8 de façon à préparer un composé de la formule

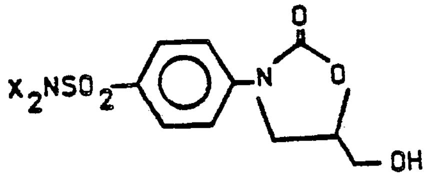

où X = Cl ou Br; ou

(ii) un sulfure de dialcoyle, $SR_8R_9$, de façon à préparer un composé de la formule

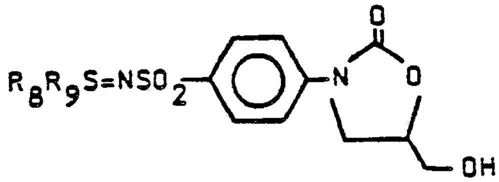

;

(d) facultativement l'oxydation du produit de l'étape (c) (ii) de façon à préparer un composé de la formule

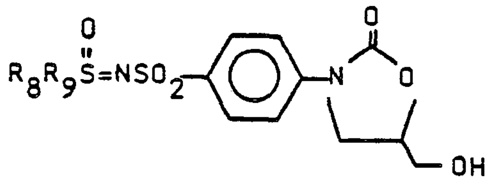

;

(e) facultativement la mise en contact du produit de l'étape (a) dans lequel $R_1 = NR_2R_3$, $R_2 = H$ et $R_3$ = un groupe alcoyle de 1—4 atomes de carbone avec un hypohalogénite de façon à préparer le composé

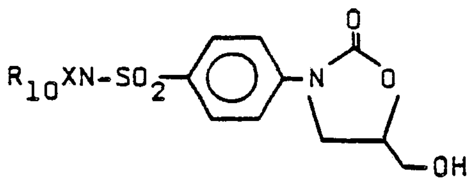

;

(f) facultativement la mise en contact du produit de l'une quelconque des étapes (a)—(e) avec
(i) un chlorure d'acide $R_6COCl$ ou un anhydride $(R_6CO)_2O$ de façon à préparer un composé de la formule

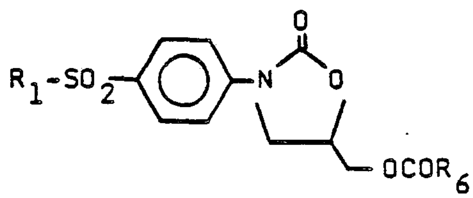

;

(ii) avec un anhydride de la formule

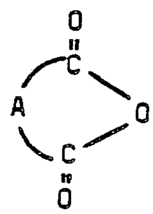

où A = $—CH=CH—$, $-(CH_2)_m—$,

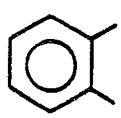 , 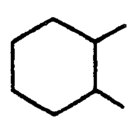 , 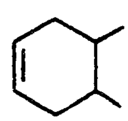 ou 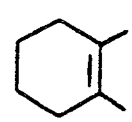 ,

et m = 2 ou 3, de façon à préparer un composé de la formule

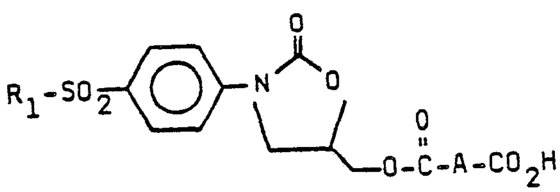

;

(iii) un amino-acide bloqué de la formule

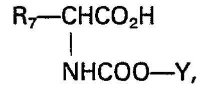

où Y est un groupe protecteur tel que le groupe *tert*-butyle, en présence d'un carbodiimide suivi d'un acide volatil, HX, de façon à préparer un composé de la formule

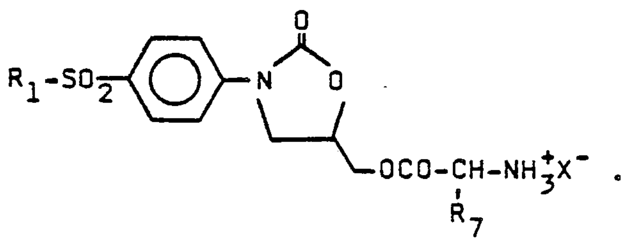

10. Un procédé pour dédoubler le 3-phénylamino-1,2-propanediol comprenant, d'une manière en elle-même connue, la mise en contact de 3-phénylamino-1,2-propanediol racémique avec environ 50 à 60 moles pour cent d'acide mandélique optiquement actif de façon qu'on obtienne un sel et la mise en contact de cel sel avec une base de façon qu'on obtienne de 3-phénylamino-1.2-propanediol enrichi et un énantiomère.

11. Le procédé selon la revendication 10, dans lequel l'acide mandélique optiquement actif est l'acide l-mandélique et le produit 3-phénylamino-1,2-propanediol est enrichi en l'énantiomère dextrogyre (R).